# EUROPEAN PATENT APPLICATION

(11) **EP 3 147 364 A1**
(43) Date of publication of application: **29.03.2017**
(21) Application number: 15382470.1
(22) Date of filing: 28.09.2015
(51) Int. Cl.: C12N 15/115, A61K 31/7088, C07H 21/00, A61P 31/00

(54) **ANTIVIRAL AGENTS COMPRISING AN OLIGONUCLEOTIDE-LIPID CONJUGATE FORMING G-QUADRUPLEX**

(71) Applicant: Institut d'Investigacions Biomédiques August Pi i Sunyer (IDIBAPS), 08036 Barcelona (ES); Consejo Superior De Investigaciones Científicas, 28006 Madrid (ES); Centro de Investigación Biomédica en Red, 28029 Madrid (ES); Universitat Pompeu Fabra, 08002 Barcelona (ES); Institució Catalana de Recerca i Estudis Avançats, 08010 Barcelona (ES)
(72) Inventor: SÁNCHEZ-PALOMINO, Sonsoles, 08018 Barcelona (ES); MIRAMBEAU, Gilles, 08036 Barcelona (ES); LYONNAIS, Sébastien, 08036 Barcelona (ES); ÁLVAREZ FERNÁNDEZ, Carmen, 08006 Barcelona (ES); ERITJA CASADELLÁ, Ramón, 08034 Barcelona (ES); GRIJALVO TORRIJO, Santiago, 08034 Barcelona (ES); MEYERHANS, Andreas, 08003 Barcelona (ES); KOUTSOUDAKIS, Georgios, 08036 Barcelona (ES); DIEZ ANTÓN, Juana, 08003 Barcelona (ES); FLETA SORIANO, Eric, 08003 Barcelona (ES); MARTÍNEZ VESGA, Javier, 08003 Barcelona (ES)
(74) Representative: ABG Patentes, S.L.

(57) **Abstract**

The present invention belongs to the field of virology and is related to agents with antiviral activity against enveloped viruses, particularly anti-HIV and anti-HCV activity, and methods for the prevention of viral infection in a subject, in particular HIV and HCV infections. Agents according to the invention comprise a DNA-lipid conjugate forming a G-quadruplex structure.

## Description

### FIELD OF THE INVENTION

The present invention belongs to the field of virology and is related to agents with antiviral activity, and methods for the prevention and/or treatment of viral infection in a subject. Agents according to the invention comprise a G-quadruplex oligonucleotide and a lipophilic moiety.

### BACKGROUND OF THE INVENTION

Enveloped viruses are a ubiquitous class of human pathogens with infection and mortality rates that can be expressed as measurable percentages of the entire human population. This class includes many well-known viruses such as influenza virus, human immunodeficiency virus (HIV), hepatitis C virus (HCV), small pox virus, chicken pox virus, yellow fever virus, herpes viruses, measles virus and many more. It also includes tropical pathogens of significant and growing global public health concern (e.g. dengue virus, Lassa fever virus and chikungunya virus), viruses that have recently elicited fears of novel and deadly global pandemics (e.g. avian influenza, SARS and MERS viruses) and viruses with significant bio-threat potential (e.g. Ebola virus, hantaviruses, Rift Valley fever virus, and most of the viruses mentioned above).

HIV infection and the related acquired immunodeficiency syndrome (AIDS) are major global health problems, with approximately 34 million people worldwide currently living with HIV and with a global HIV prevalence expected to increase within the next 10 years.

There are 6 major classes of treatments containing about 32 antiretroviral (ARV) products approved for the treatment of the HIV-1/AIDS pandemic. The majority of drugs belong to the reverse transcriptase inhibitor and protease inhibitor classes. Combinations of ARV drugs were developed as "multiclass" products, but the available combinations are limited, lead to resistance, often provide severe side effects and are still costly. Hence, there is a need for development of new anti-HIV therapeutic agents based on other targeting strategies including multimodal activity in a single compound. Also of a crucial importance is the research of methods and means capable of stopping HIV particles before their entry in the cytoplasm of the targeted cell, as an alternative to AIDS vaccine or combined with the emerging strategy of AIDS mucosal vaccines. For example, maraviroc is an HIV entry inhibitor available in the market that inhibits the viral Envelope Glycoprotein (EnvGP) binding to the cellular CCR5 co-receptor.

Sexual intercourse remains the predominant route of HIV-1 transmission worldwide, emphasizing a clear and urgent need for additional prevention strategies, including the development of microbicides, i.e., mucosally applied products designed to prevent HIV infection. Microbicides are topical products, such as gels, capsules, tablets, films, and intra- vaginal rings. They are designed to be applied either around the time of coitus, used on a daily basis (gels and films), or to deliver product over a prolonged period of time. The premise is inhibition or blockade of the earliest steps in the infection process at the vaginal or rectal mucosa. Because microbicides are topical, higher local drug concentrations can be delivered to virally exposed surfaces without significant systemic exposure, thereby reducing the risk of long-term toxicity in healthy but at-risk individuals. There have been nine fully-completed or halted trials of microbicide candidates, one of which showed that the prototype actually boosted the risk of infection by causing vaginal lesions that helped the virus to enter the bloodstream. The new tack is to benefit from the experience acquired with conventional HAART regiments to produce microbicide compositions that incorporate combination of ARV drugs with different mechanisms of action, targeting different stage of the viral replication cycle (especially viral entry), and already tried-and-tested among people with HIV. At least five gels in this category are in the early stages of trials and there is a true cause for optimism in this field, as showed by the promising results from a phase IIb clinical trial, CAPRISA 004, of a vaginal microbicide based on the reverse transcriptase inhibitor tenofovir.

HCV is the major causative agent of non-A, non-B viral hepatitis. It is the major cause of transfusion-associated hepatitis and accounts for a significant proportion of hepatitis cases worldwide. Although acute HCV infection is often asymptomatic, nearly 80% of cases develop chronic hepatitis. The persistent property of the HCV infection has been explained by its ability to escape from the host immune surveillance through hypermutability of the exposed regions in the viral envelope glycoprotein E2.

HCV treatment efficacy is influenced by viral genotype. Specifically, interferon-based therapy regimes attain sustained virological response (SVR) rates of approx. 80% in patients with genotype 2 and 3 infections, while SVR rates among patients chronically infected with genotype 1 viruses drop to 50%. Although the introduction of protease inhibitors to the current standard-of-care interferon/ribavirin therapy has improved the sustained virological response of genotype 1-infected patients, these inhibitors exacerbate already problematic side effects. A recently approved nucleoside analogue, sofosbuvir, used in combination with daclatasvir achieved a sustained viral response at 24 weeks without added interferon against all HCV genotypes. These last treatments are promising but remain very costly and clinicians are now facing problems with viral resistance.

Oligonucleotides and DNA aptamers which bind to HIV EnvGP gp120 have been described and show significant antiviral activity by blocking the steps of virus attachment and/or entry in the host cell by interfering with the viral EnvGP binding to its cellular receptors or co-receptors (Oliviero G et al. 2010 Chem Commun 46: 8971-8973; D'Atri V et al. 2012 Chem Commun 48: 9516-9518). Antiviral agents based on candidate molecules identified by interaction with G-quadruplex structures formed in the central flap structure of HIV-1 DNA have been suggested (US 200610183106 A1). Microbicide compositions comprising a oligonucleotides forming G-quadruplex structures for the prevention and treatment of retrovirus associated infectious diseases have been described (WO 2010/133652 A1). Potential anti-HIV agents based on G-quadruplex comprising the Hotoda sequence (5'-TGGGAG-3') have been proposed (Musumeci & Montesarchio, 2012, Molecules 17:12378-92).

The HIV-1-derived sequence (5'-TTGGGGGGTACAGTGCA-3') (SEQ ID NO: 3) is known to adopt a G-quadruplex structure (Lyonnais S et al. 2003 Nucleic Acids Res 31: 5754-63). However, no antiviral activity has been described for this sequence.

On the other hand, oligonucleotides forming G-quadruplex structures have not been developed for the treatment of HCV infection.

A large number of oligonucleotides have been modified with membrane anchors to modulate their biological activity, by increasing their binding affinity towards cell membranes. In selecting a lipophilic, and particularly a cholesteryl, derivative with optimal features for terminal modifications oligonucleotides forming G-quadruplex, several issues have to be considered: water solubility problems, chemical stability and hydrophobicity-driven cholesterol self-assembly that could hamper, for steric reasons, the correct G-quadruplex formation. However, while the addition of cholesterol as a conjugating agent has been well studied for oligonucleotides, its contribution as a derivate in G-quadruplex structures has been poorly explored. An aspect of cholesterol is its steric hindrance, which may be detrimental to G-quadruplex formation, particularly in the case of parallel structures. Wolfe and Goodchild demonstrated that the anti-HIV activity of G-rich oligonucleotides can be dramatically influenced, either positively or negatively, by the presence of cholesteryl groups, with enhanced bioactivity only if the hydrophobic groups are covalently attached at positions distant from the G-quartets (Wolfe & Goodchild, J. Am. Chem. Soc. 1996; 118:6301-6302). Recently, Musumeci and Montesarchio presented 5'- conjugates of the anti HIV-1 Hotoda sequence linked with cholesterol moieties (Musumeci & Montesarchio, 2012, Molecules 17:12378-92). They disclosed the correct G-quadruplex assembly of the cholesterol-modified oligonucleotide by HPLC and mass spectrometry and report studies in progress to evaluate the antiviral activity of these new compounds.

All the reasons above underline the need to discover new antiretroviral drugs and to propose potentially new therapeutic way of HIV and/or HCV inhibition. Thus, despite the efforts made to date, there still exists a long-felt and continuing need in the art for reliable antiretroviral drugs.

### SUMMARY OF THE INVENTION

In a first aspect, the invention relates to an agent comprising one unit, two units or four units, wherein each of said units comprises:
(i) an oligonucleotide comprising at least four consecutive guanines (G), wherein said at least four consecutive G form an intra-unit (intra-molecular) or an inter-unit (inter-molecular) G-quadruplex, wherein said G-quadruplex binds to a viral envelope glycoprotein,
and wherein at least one of said units comprises:
(ii) a lipophilic substituent moiety, wherein said lipophilic molecule anchors to a viral envelope or to the membrane of a cell susceptible of being infected by a virus, and
(iii) a linker, wherein said linker links the 5' or 3' end of the oligonucleotide in (i) to the lipophilic substituent moiety in (ii),
wherein said agent has antiviral activity.

In another aspect, the invention relates to a pharmaceutical composition comprising a therapeutically effective amount of an agent according to the invention, together with at least one pharmaceutically acceptable excipient or carrier.

In a further aspect, the invention relates to the agent or the pharmaceutical composition according to the invention for use in medicine.

In a further aspect, the invention relates to the agent or the pharmaceutical composition according to the invention for use in the prevention and/or treatment of viral infection in a subject, wherein said viral infection is caused by an enveloped virus.

### DESCRIPTION OF THE FIGURES

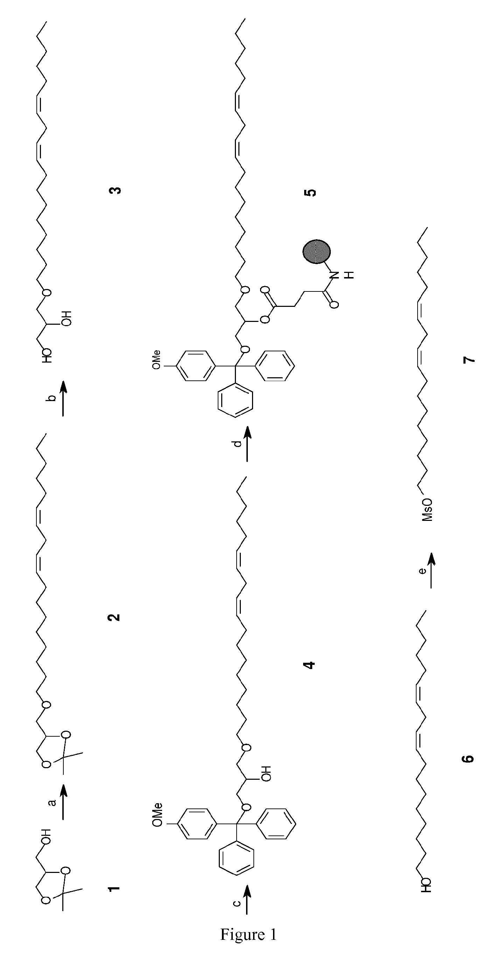

Figure 1. Synthetic route to functionalize Controlled Pore Glass solid-support with linoleic alcohol. **(1)** 2,2-Dimethyl-1,3-dioxolan-4-yl)methanol or solketal. **(2)** 2,2-dimethyl-4-{[(10*Z*,*13Z*)-octadeca-10,13-dien-1-yloxy]methyl}-1,3-dioxolane; **(3)** 3-[(*10Z,13Z*)-octadeca-10,13-dien-1-yloxy]propane-1,2-diol; **(4)** 1-[(4-methoxyphenyl)(diphenyl)methoxy]-3-[(*10Z,13Z*)-octadeca-10,13-dien-1-yloxy]propan-2-ol; **(5)** Controlled pore glass functionalized with compound **(4); (6) -** (*10Z,13Z*)-octadeca-10,13-dien-1-ol; **(7)** (*9Z,12Z*)-octadeca-9,12-dien-1-yl methylsulfonate. Reagents and conditions: a. NaH (60%); (*9Z,12Z*)-octadeca-9,12-dien-1-yl-methylsulfonate **(7),** toluene, reflux, overnight; b. *p*-methylbenzenesulfonic acid (TsOH), methanol, 5 h at room temperature; c. (4-Methoxyphenyl)(diphenyl)methyl chloride (MMT-Cl), *N,N*-dimethylaminopyridine (DMAP), Pyridine, 45 °C, overnight; d. CPG functionalization: first reaction with succinic anhydride, DMAP and then reaction with amino-controlled pore glass (CPG) by activation of hemisuccinate with triphenylphosphine, 2,2-dithiobis(5-nitropyridine) and DMAP and reaction with ; e. methylsulfonic acid chloride, Pyridine, CH₂Cl₂, room temperature, overnight.

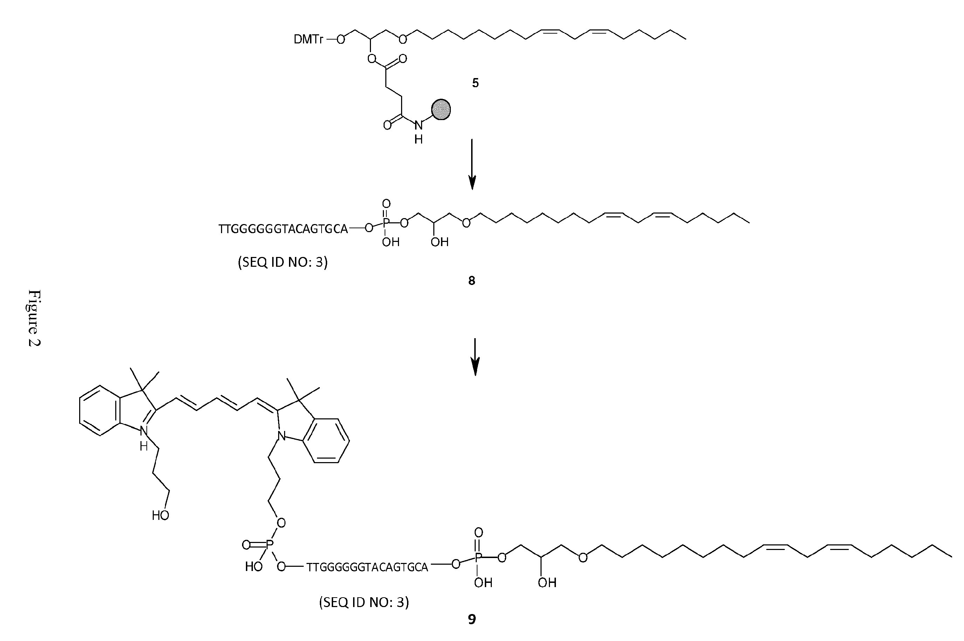

Figure 2. Synthesis of modified GQVIR17-3'C18 **(8)** and 5'Cy5-GQVIR17-3'C18 **(9).** The DNA sequence 5'-(dTTGGGGGGTACAGTGCA)-3' was assembled on controlled pore glass functionalized with 1-[(4-methoxyphenyl)(diphenyl)methoxy]-3-[(*10Z,13Z*)-octadeca-10,13-dien-1-yloxy]propan-2-yl succinate **(5)** using standard solid-phase methods. After ammonia deprotection GQVIR17-3'C18 **(8)** was obtained. The same protocols were used for the synthesis **(9)** 5'Cy5-GQVIR17-3'C18 but in this case the phosphoramidite of Cy5 was used to incorporate this label at the 5'-end of the sequence.

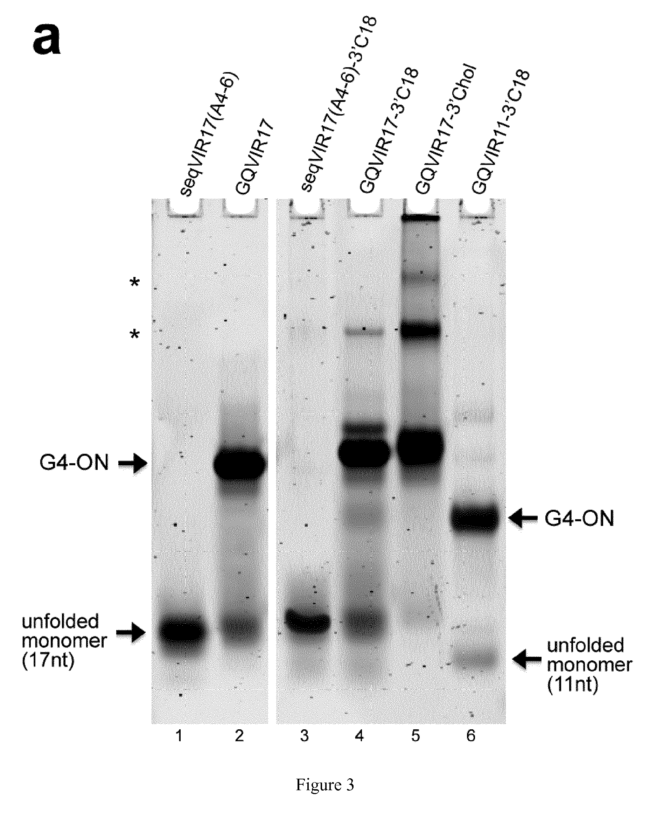

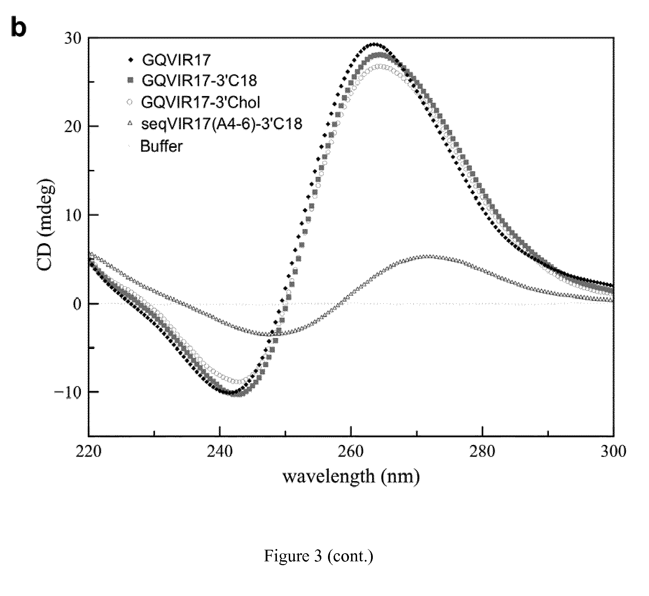

Figure 3. ODNs used in this study and G4-ON formation. (a) PAGE separation of the indicated ODNs after two weeks of incubation at room temperature in 20mM Tris-Acetate (pH 7.0), 50mM Potassium Acetate. 12% non-denaturing poly-acrylamide gels were run at 100V for 2h, stained with SYBr Gold and digitalized with a bioimaging system. ODNs forming tetramolecular species (G4-ON) appear as a retarded, shifted band indicative of higher molecular weight species, as seen in Lyonnais et al, 2002 (Nucl Acids Res 30:5276-83). (*) high order species (presumably dimers) observed for GQVIR17-3'C18 and -3'Chol. (b) CD Spectra at 20°C in 10mM sodium cacodylate (pH 6.8) and 50mM KCl of GQVIR17 (black diamonds), GQVIR17-3'C18 (grey squares), GQVIR17-3'Chol (circles) and seqVIR17(A'-6)-3'C18 (triangles) after two weeks of incubation in 50mM KCl.

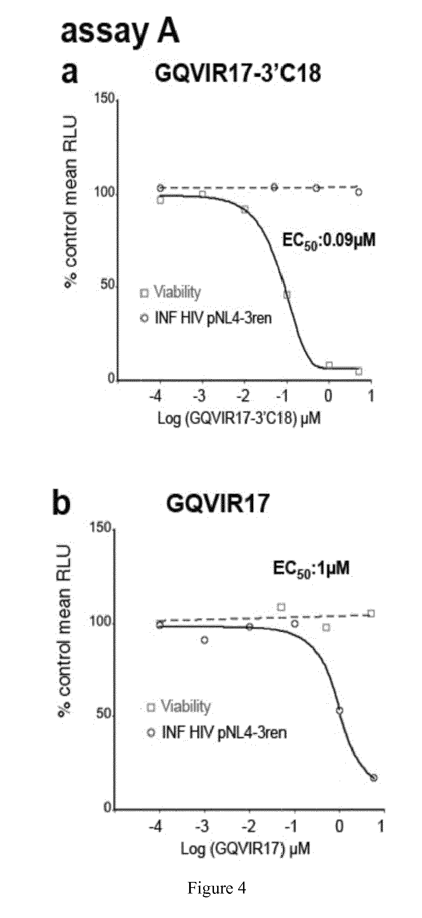

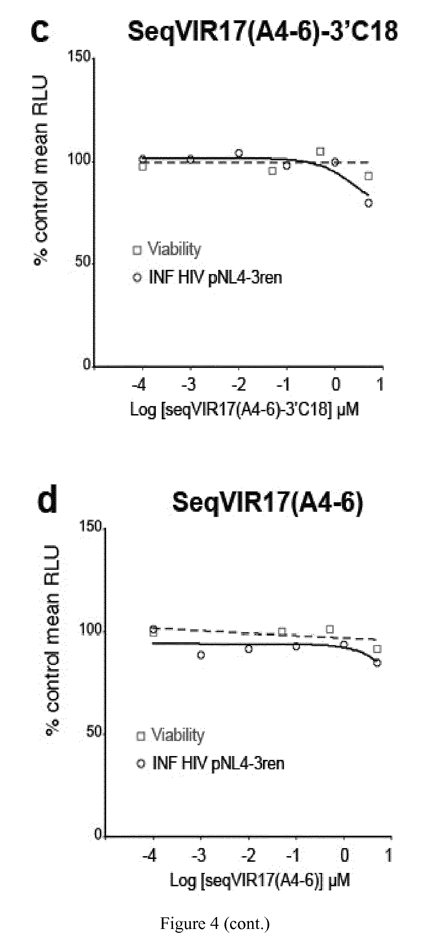

Figure 4. Inhibition of HIV-1 infection and viability. Anti-HIV activity and viability of SupT1 cells infected or not with NL4.3-Ren and treated for 72 h with different concentrations of the indicated drugs. Concentration-response curves are shown as percentage of RLUs as compared to a non-treated control.

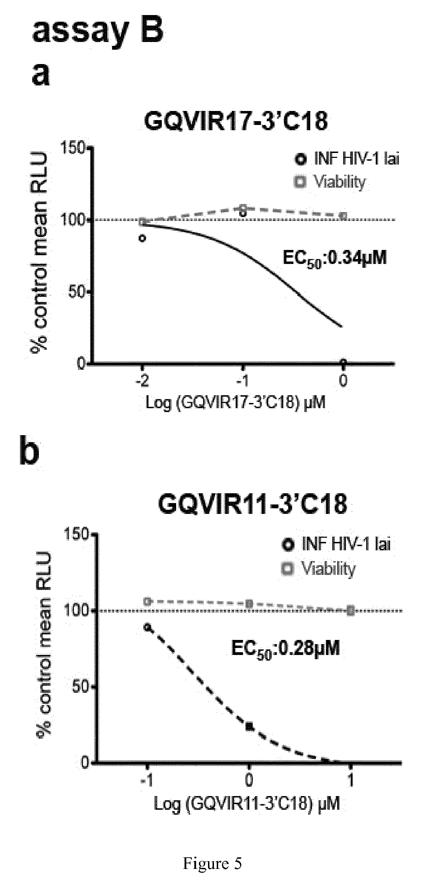

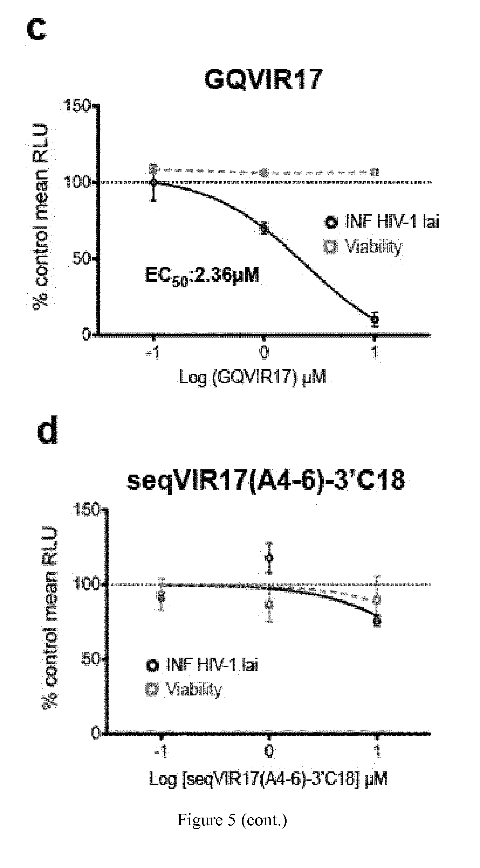

Figure 5. Inhibition of HIV-1 infection and viability. Anti-HIV activity and viability of TZM-bl cells infected with HIV-1 Lai and treated with different concentrations of the indicated drugs. Concentration-response curves are shown as percentage of RLUs as compared to a non-treated control.

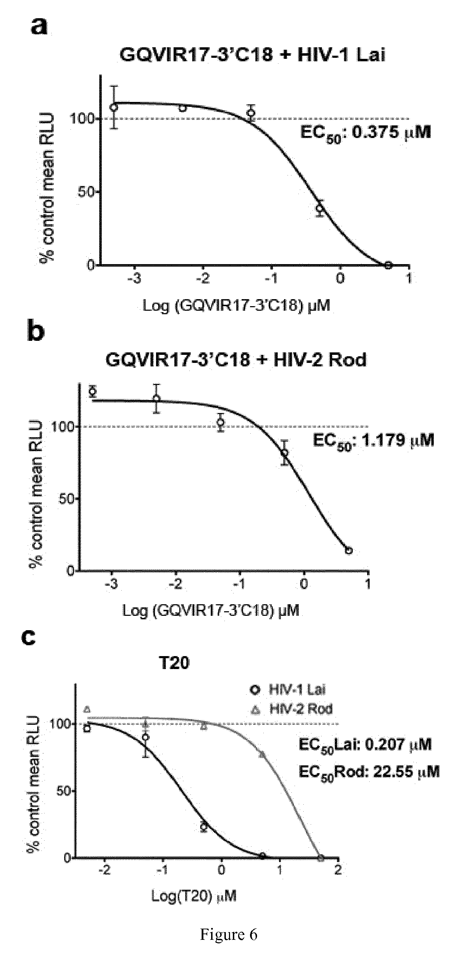

Figure 6. Inhibition of HIV-1 and HIV-2 infection. Anti-HIV activity and viability on TZM-bl cells infected with HIV-1 Lai or HIV-2 Rod and treated with different concentrations of the indicated drugs. Enfuvirtide, a fusion inhibitor (T20), was used as a control.

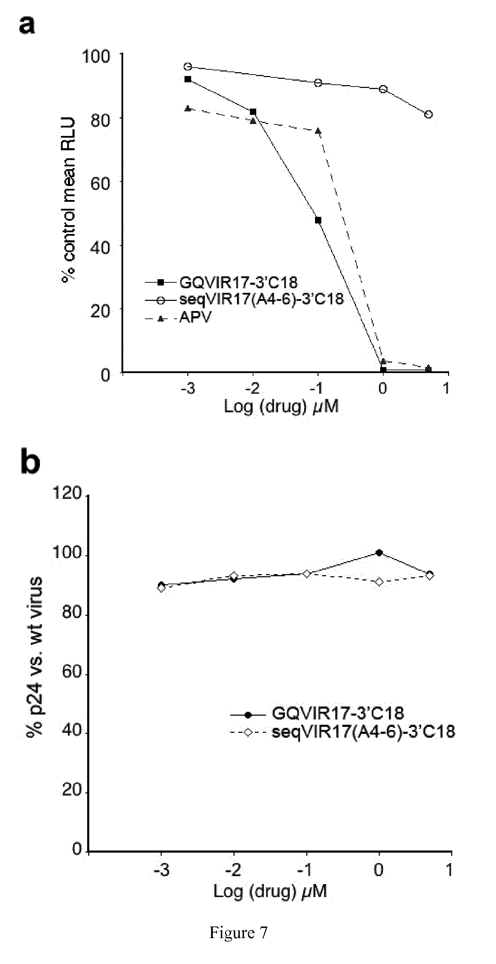

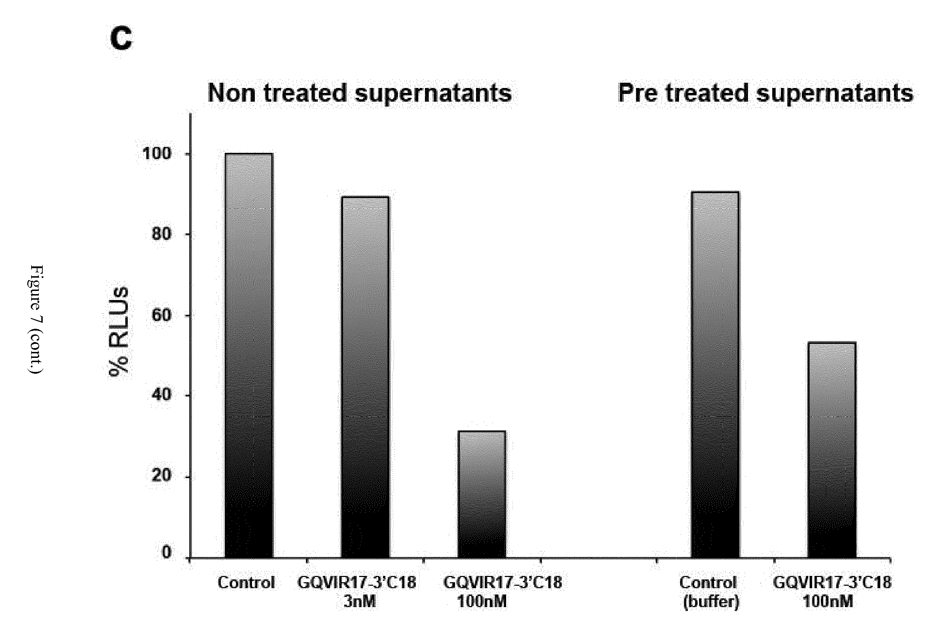

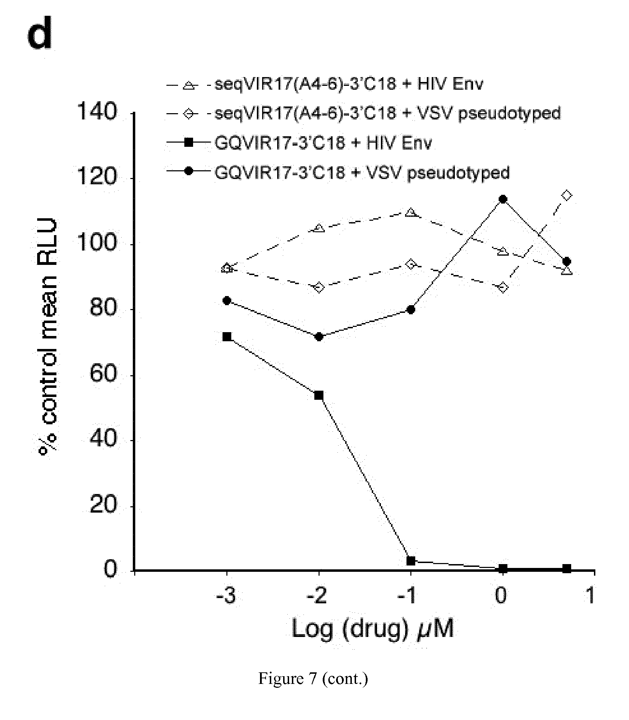

Figure 7. GQVIR17-3'C18 inhibits HIV entry. (a) Luciferase dose-response curves for NL4.3-Ren viruses in SupT1 cells. NL4.3-Ren viruses were produced in 293T cells treated 24h in presence of drugs. Results are shown as percentage of RLUs as compared to a non-treated control. (b) Quantification of p24 and percentage of NL4.3-Ren viruses produced in 293T cells in the presence of drugs as compared to a non-treated control. (c) Inhibition of HIV infection by GQVIR17-3'C18 in SupT1 cells infected with non-treated or pre-treated supernatants obtained from transfections of pNL4.3-Ren in 293T cells. (d) Anti-HIV activity on SupT1 cells infected with NL4.3-ren or NL4.3-Δenv-VSV-Luc and treated for 48 h with different concentrations of drugs.

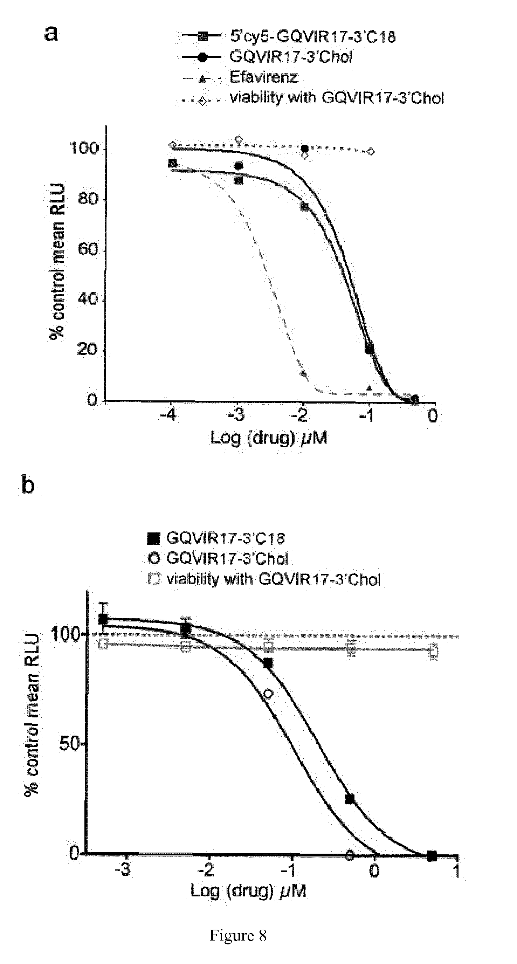

Figure 8. Antiviral activity of GQVIR17-3'C18 and GQVIR17-3'Chol (a) infectivity assay A with NL4.3 and SupT1 cells treated with the indicated compounds. (b) infectivity assay B with HIV-1 Lai and TZM-bl cells treated with the indicated compounds.

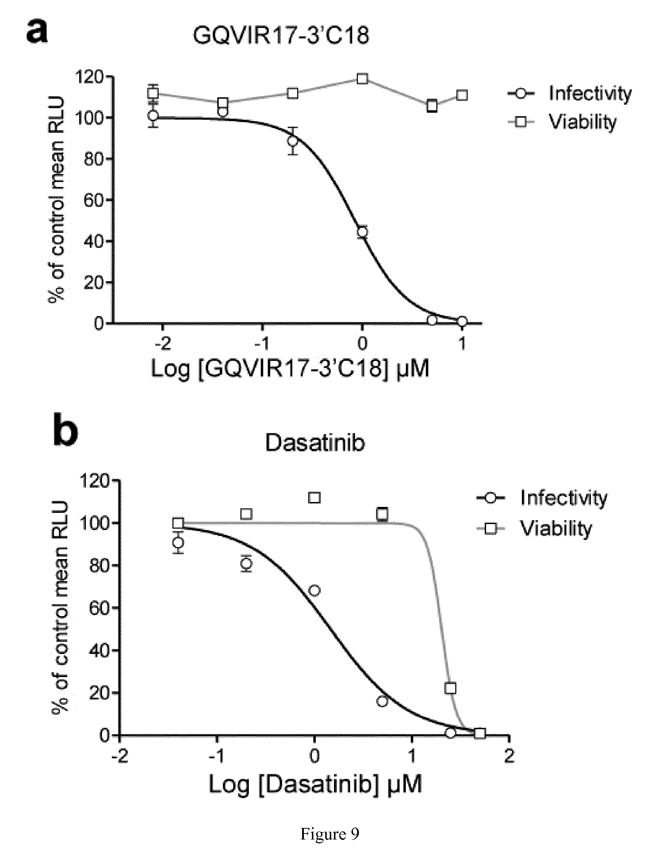

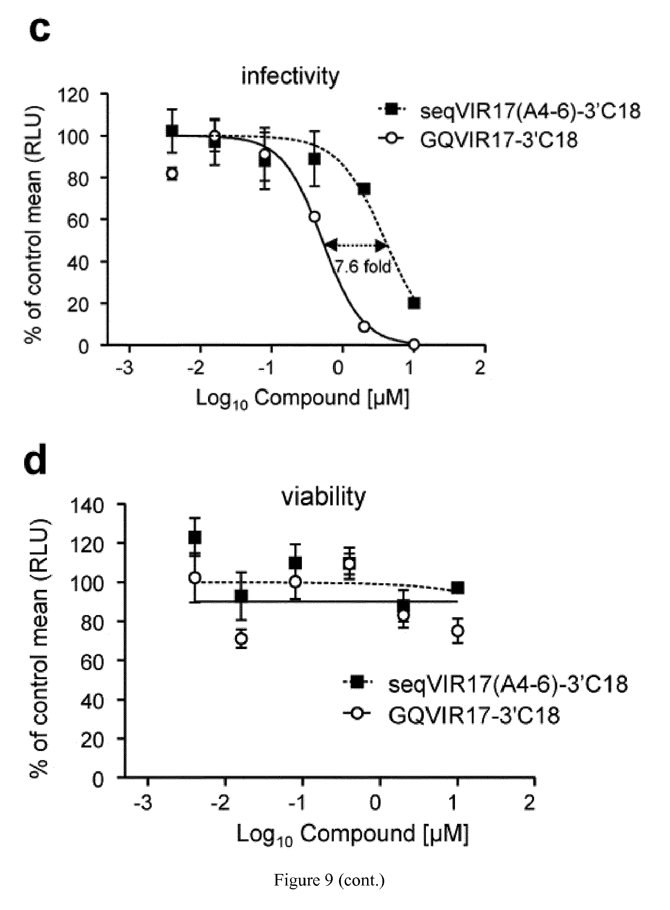

Figure 9. GQVIR17-3'C18 inhibit HCV genotype 2a in a specific manner. Anti-HCV activity and viability on Huh7/Scr cells infected with Luc-Jc1 viruses and treated with increasing concentrations of GQVIR17-3'C18 (a), Dasatinib (b) and GQVIR17-3'C18 or seqVIR17(A4-6)-3'C18 (c: infectivity, d: viability). Virus-compounds mix were replaced 4 h post infection with fresh media-compounds mix and 72 h after infection cells were assayed for Firefly luciferase activity and the mean relative light units (RLU) were plotted as percentage relative to DMSO for both infectivity and cell viability.

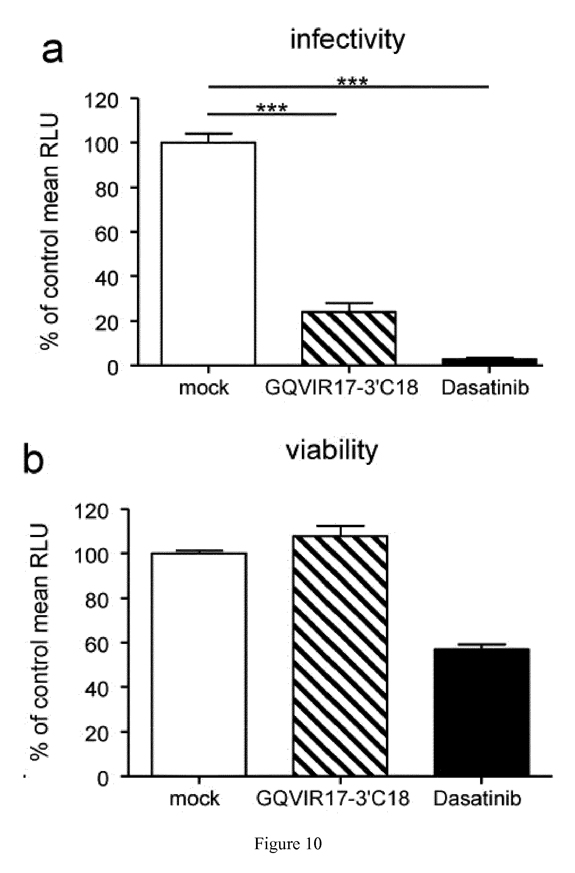

Figure 10. GQVIR17-3'C18 inhibit HCV pseudoparticles (HCVpp) cell entry. Huh7/Scr cells were seeded on 96 well plates, 1.2 x 104 cells/well, 16 h prior to infections. The day of infections, cells were treated with 5µM of GQVIR17-3'C18 or Dasatinib for 1 h. Then, compounds-containing media were removed and cells were infected with HCVpp-compounds mix at the same concentration. HCVpp-compounds mix were replaced 6 h post infection with fresh media-compounds mix and 72 h after infection cells were assayed for Renilla luciferase activity and the mean relative light units (RLU) were plotted as percentage relative to DMSO for both infectivity and cell viability. The data presented are from a single experiment and are representative of 3 independent experiments. Data are expressed as mean values of quadruplicates (± SEM).

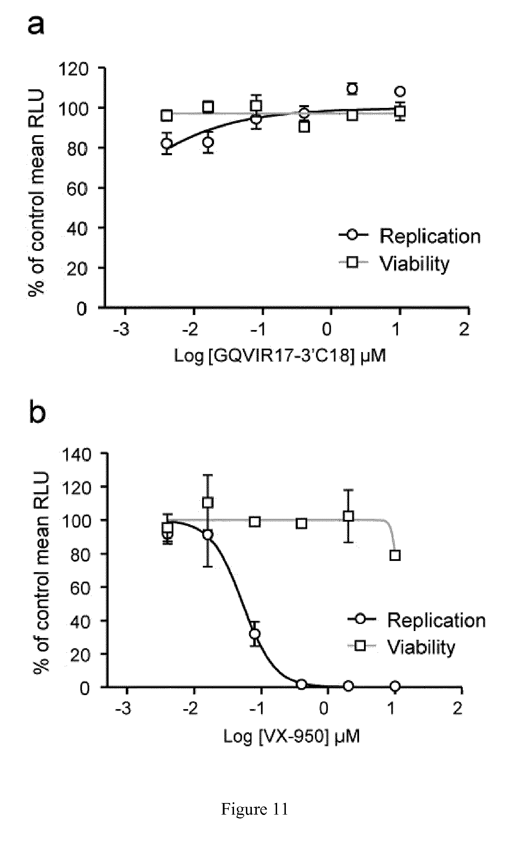

Figure 11. GQVIR17-3'C18 do not inhibit HCV translation and/or replication. Huh7/Scr cells were transfected with the sub-genomic luciferase replicon by electroporation and seeded on 96 well plates, 1.2 x 104 cells/well. Drugs were added 4 hours after transfection and the level of HCV-RNA translation and RNA replication was quantified 48 hours later. The mean relative light units (RLU) were plotted as percentage relative to buffer or DMSO for GQVIR17-3'C18 or VX-950, respectively, for both infectivity and cell viability. The data presented are from a single experiment and are representative of 3 independent experiments. Data are expressed as mean values of quadruplicates (± SEM).

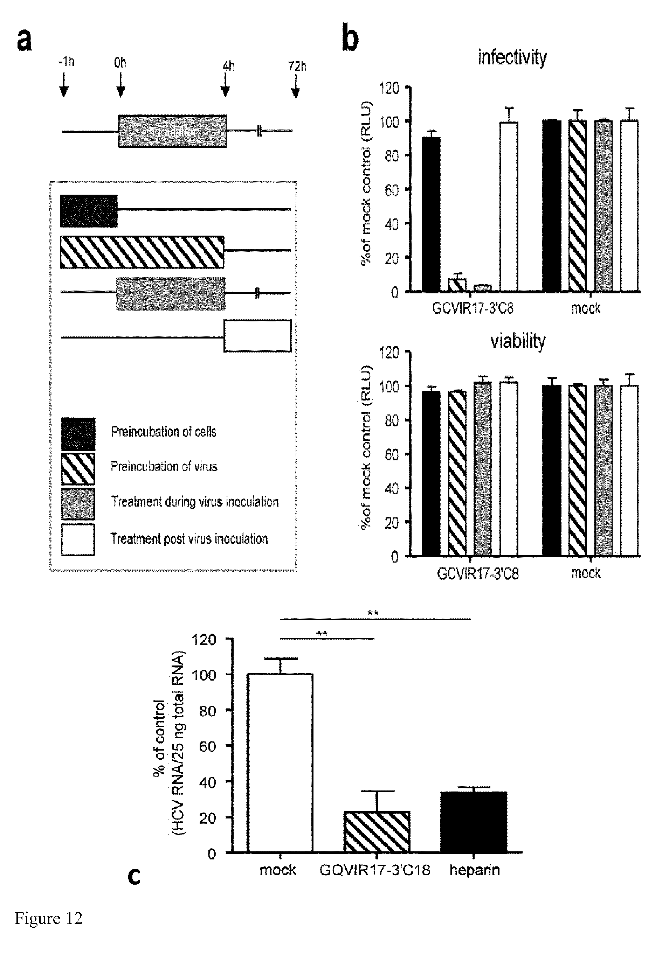

Figure 12. GQVIR17-3'C18 interacts with HCV particles, selectively inhibits early HCV entry events and inhibits the attachment of HCV to the surface of Huh7/Scr cells. Huh7/Scr cells were inoculated with Luc-Jc1 viruses prepared in the absence of drugs. GQVIR17-3'C18 (final concentration 1 µM) were added to the cells only before inoculation for 1h (black), pre-incubated with the viruses (stripes), only during inoculation (grey) or selectively after infection, as schematically depicted in (a). Infectivity was determined 72 hours later by luciferase assays and the mean relative light units (RLU) were plotted as percentage relative to buffer for both infectivity and cell viability. (c) Huh7/Scr cells were placed on ice for 30 min to cool down. Then cells were inoculated with cold Jc1 viruses at an MOI ∼10 TCID₅₀/cell at 4°C. GQVIR17-3'C18 (final concentration 1 µM) or heparin (100 µg/ml) were added to the cells simultaneously to inoculation. After 2h, cells were extensively washed with ice-cold PBS and bound HCV was determined with a specific HCV RT-qPCR. HCV RNA is plotted as percentage relative to buffer (mock). The data presented are from a single experiment and are representative of 3 independent experiments. Data are expressed as mean values of quadruplicates (± SEM).

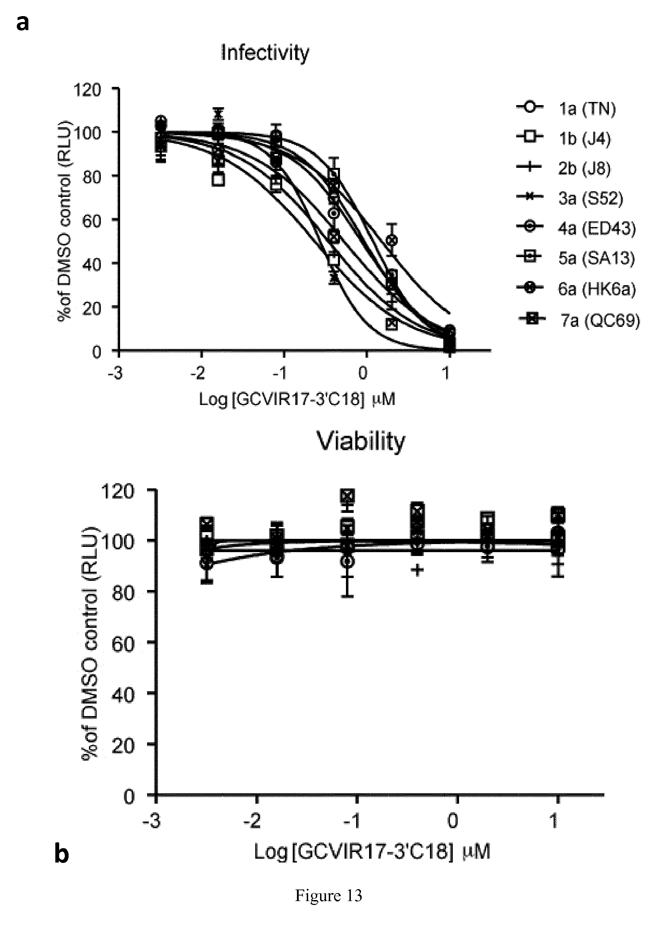

Figure 13. GQVIR17-3'C18 inhibits HCV of different genotypes. Huh7/Scr cells were inoculated with viruses of the indicated genotypes and isolates at an MOI ∼0.01-0.03 TCID₅₀/cell. GQVIR17-3'C18 in the indicated concentrations was added to the cells simultaneously to inoculation. HCVcc-compounds mix were replaced 4 h post infection with fresh media-compounds mix. 72 h after infection cells were assayed for Renilla luciferase activity (a) and for cytotoxicity (b). The mean relative light units (RLU) were plotted as percentage relative to buffer (mock) for both infectivity and cell viability. The data presented are from a single experiment and are representative of 3 independent experiments. Data are expressed as mean values of quadruplicates (± SEM).

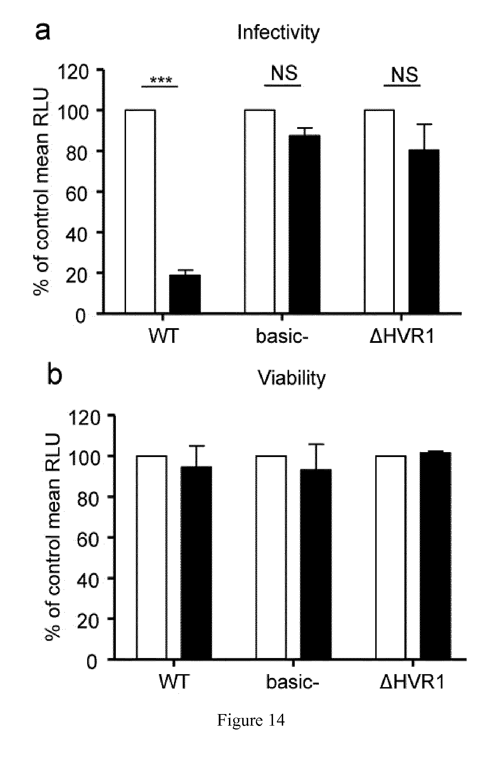

Figure 14. GQVIR17-3'C18 inhibits HCV of different genotypes. Huh7/Scr cells were inoculated with Luc-Jc1 WT viruses or with the indicated mutants at an MOI ∼0.01-0.03 TCID₅₀/cell. GQVIR17-3'C18 (final concentration 1 µM) was added to the cells simultaneously to inoculation. HCVcc-compounds mix were replaced 4 h post infection with fresh media-compounds mix and 72 h after infection cells were assayed for Firefly luciferase activity and the mean relative light units (RLU) were plotted as percentage relative to buffer (mock) for both infectivity and cell viability. The data presented are from a single experiment and are representative of 3 independent experiments. Data are expressed as mean values of quadruplicates (± SEM).

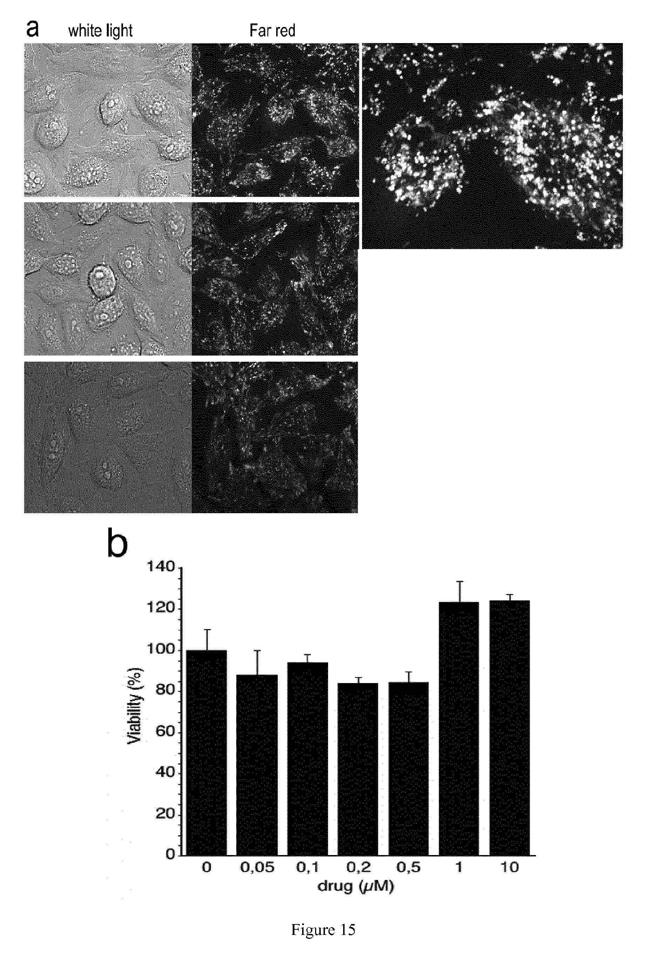

Figure 15. Visualization of 5'Cy5-GQVIR17-3'C18 in HeLa cells and viability. (a) Images of HeLa cells treated with 1 µM of 5'Cy5-GQVIR17-3'C18 for 4 h at 37°C under white and far red light. 40 000 HeLa cells were seeded and cultured overnight. After washing with culture medium, cells were treated with 1 µM of 5'Cy5-GQVIR17-3'C18 for 4 h at 37°C. Then, the medium was replaced with DMEM without Phenol Red and cells were observed under a Zeiss Axio Observer.Z1 microscope equipped with a 37°C incubation room. Images were taken with a OrcaR2 (Hamamatsu) camera, using a far red filter (EX BP 640/30, EM BP 690/50). Acquisitions were made with the AxioVision4.8 software and presented images are the projections of all z stacked signals (0,230 µm between each images), analysed with the ImageJ software. (b) Viability assay at 48h as function of drug concentration. 9 500 cells/well were seeded in 96-well plates and cultured overnight in DMEM with 7% FCS. Then, cells were washed with PBS and treated with indicated concentrations of GQVIR17-3'C18 conjugate in 50 µL optiMEM (Gibco) for 4 hours. Thereafter, 150 µL of medium with 7% FCS was added and 24 and 48 hours after the viability of cells was measured using the CellTiter 96 Aqueous Non-radioactive Cell Proliferation Assay (MTS-assay, Promega) according to the manufacturer's instructions.

### DETAILED DESCRIPTION OF THE INVENTION

The authors of the present invention have developed an agent capable of inhibiting viral entry into the host cell. The agent of the invention comprises an oligonucleotide (ODN) that binds subdomains of the viral envelope glycoproteins (EnvGP) and a lipophilic group that anchors the agent to the membrane of a viral particle and/or to the membrane of a cell.

Particularly, the authors of the present invention have developed and characterized an ODN covalently linked with lipid groups and capable of forming highly stable secondary structures termed G-Quadruplex. This lipid-labelled G-quadruplex (termed as LIPOQUAD by the inventors) efficiently targets HIV gp120 EnvGP and HCV E2 EnvGP due to a designed-membrane attachment at the surface of the target cell, and/or a specific attachment on the viral particles. These lipid-labelled G-quadruplexes are thought to operate in a novel fashion at an early stage of viral infection, by inhibiting entry of the viral particle into a cell (e.g. *in vitro* and *in vivo*) comprising contacting the cell or/and the virus with the oligonucleotide-lipid conjugate, as shown in Examples 2 and 3, which should provide a persistent protection of the mucosal tissues exposed to HIV or/and HCV viral particles due to the virucidal activity of the agent of the invention.

An important key point of this agent is also the biochemical stability given by the G-quadruplex structure and prolonged half-life. Unmodified, these G-Quadruplex DNA structures present very high melting temperatures (>90°C) and are resistant to nucleases, a property enhanced by the conjugation with a lipid, which protects the most sensitive part of the molecule (the single-stranded tails). In addition, the lipid-labelled G-quadruplexes of the invention withstand varying buffers, storage conditions and pH (exposed to significant variations in the vagina) and present very low cytotoxicity. Furthermore, it is not expected to eliminate the natural beneficial lactobacilli that reside in the vagina and regulate vaginal health. These findings should clearly represent a major breakthrough for treating viral infection.

### Definitions

The term "alkyl" refers, in the present invention, to linear or branched hydrocarbon chain radicals which are attached to rest of the molecule by means of a single bond, for example, methyl, ethyl, n-propyl, i-propyl, n-butyl, tert-butyl, sec-butyl, n-pentyl, n-hexyl, etc.

The term "capping group", as used herein, and also known as "protecting group" or "cap structure", shall be understood to mean chemical modifications, which have been incorporated at either terminus of the oligonucleotide comprised by the agent according to the invention. In particular, said capping group is linked to the end of the oligonucleotide comprised by the agent of the invention which is not attached to the linker comprised by said agent. Non-limiting examples of the 5'-cap include inverted abasic residue (moiety), 4',5'-methylene nucleotide; 1 -(beta-D-erythrofuranosyl) nucleotide, 4'-thio nucleotide, carbocyclic nucleotide; 1,5-anhydrohexitol nucleotide; L-nucleotides; alpha- nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety. Details are described in WO97/26270, incorporated by reference herein. The 3'-cap includes, for example, 4',5'-methylene nucleotide; 1 -(beta-D-erythrofuranosyl) nucleotide: 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inveiled abasic moiety; 5'-phosphoramidate; 5'-phosphorothioate; 1,4-butanediol phosphate; 5 '-amino; bridging and/or non-bridging 5'-phosphoramidate, phosphorothioate and/or phosphorodithioate, bridging or non-bridging methylphosphonate and 5'-mercapto moieties. See also Beaucage and Iyer, 1993, Tetrahedron 49, 1925; the contents of which are incorporated by reference herein. Preferred protecting groups according to the invention are selected from the group comprising poly-L-lysine, propylamine, biotine and a fluorescent dye. Fluorescent dyes according to the invention include, without limitation, acridine dyes, cyanine dyes, fluorine dyes, oxazin dyes, phenanthridine dyes and rhodamine dyes. In a particular embodiment, the capping group linked to the end of the oligonucleotide comprised by the agent of the invention which is not attached to the linker is selected from the group comprising poly-L-lysine, propylamine, biotine and a fluorescent dye.

The expression "cell susceptible of being infected by a virus" or "cell susceptible of infection", as used herein, relates to any cell susceptible of being infected by a virus, that is to say, a cell which holds a protein receptor that the Envelope Glycoprotein embedded in the membrane bilayer of the virus can recognize and bind to. The envGP of a virus becomes connected to complementary receptors, termed co-receptors, on the cell surface susceptible of infection by said particular virus. In the case of enveloped viruses, the interaction between the EnvGP and the cellular receptor and the co-receptors leads to a fusion of the viral and cell membranes, which ultimately drives virus entry in the host cell.

The term "cellular receptor", as used herein, relates to host proteins embedded in the membrane of a cell susceptible of infection by a virus and recognised and bound by the viral Envelope Glycoproteins of said virus. The EnvGP of the virus can bind to complementary co-receptors on the cell membrane. Cellular receptors may facilitate virus attachment, virus internalization and/or transduction of intracellular signals following virus binding. The term "host cell co-receptor", as used herein, relates to those co-receptors proteins located on the cell membrane of a cell susceptible of being infected by a virus, wherein said co-receptors are bound by the EnvGP located at the viral envelope of said virus.

The term "composition", as used herein, relates to a material composition that comprises at least two components, as well as any product resulting, directly or indirectly, from the combination of the different components in any quantity thereof. Those skilled in the art will observe that the composition may be formulated as a single formulation or may be presented as separate formulations of each of the components, which may be combined for joint use as a combined preparation. The composition may be a kit-of-parts wherein each of the components is individually formulated and packaged.

The term "enveloped virus", as used herein, refers to any animal virus, which possesses an outer Envelope Glycoprotein embedded in a lipid bilayer surrounding the inner part of a virion. Exemplary enveloped viruses include, but are not limited to, members of the *Herpesviridae, Poxviridae, Hepadnaviridae, Togaviridae, Arenaviridae, Flaviviridae, Orthomyxoviridae, Paramyxoviridae, Bunyaviridae*, *Rhabdoviridae*, *Filoviridae, Coronaviridae, Retroviridae* and *Bornaviridae* virus families.

The term "detectable tag", as used herein, also known as "detectable compound", relates to any compound which can be detected either directly due to any property of said compound or indirectly because said compound has the capacity to modify a second molecule which is detectable. The compound can be a fluorescent dye, a luminescent group or an enzyme. If the detectable compound is an enzyme, then this enzyme must be capable of generating a detectable signal, for example, after adding an activator, substrate, amplifying agent and the like.

The expression "disease associated with a HIV infection", as used herein, includes a state in which the subject has developed AIDS, but also includes a state in which the subject infected with HIV has not shown any sign or symptom of the disease.

The expression "disease associated with an infection caused by an enveloped virus", as used herein, includes without limitation:
- Diseases caused by enveloped viruses belonging to the herpes viruses family, such as varicella and herpes zoster (caused by varicella zoster virus (VZV)), herpes labialis and genital herpes (caused by herpes simplex virus type 1 and 2 (HSV-1 and HSV-2)), Marek's Disease (MD) in chickens caused by Marek's Disease Virus (MDV), infectious mononucleosis, Burkitt's lymphoma, CNS lymphoma in AIDS patients, post-transplant lymphoproliferative syndrome (PTLD), nasopharyngeal carcinoma and HIV-associated hairy leukoplakia caused by Epstein-Barr virus, Infectious mononucleosis-like syndrome and retinitis caused by cytomegalovirus, Sixth disease caused by Roseolovirus and Kaposi's sarcoma caused by human herpesvirus-8,
- Diseases caused by enveloped viruses belonging to the *Filoviridae* family including, without limitation, Marburg virus disease (Marburg hemorrhagic fever or MHF), caused by Marburg virus and Ebola virus disease (Ebola Hemorrhagic Fever or EHF), caused by Ebola virus,
- Diseases caused by an enveloped virus belonging to the *Retroviridae* family, and
- Other diseases caused by an enveloped virus infection include, without limitation, Dengue fever, Dengue hemorrhagic fever (DHF), yellow fever, dengue fever, acute and chronic hepatitis C, Venezuelan hemorrhagic fever, Brazilian hemorrhagic fever, Bolivian hemorrhagic fever, lymphocytic choriomeningitis, Lassa fever, hantavirus pulmonary syndrome (HPS), meningitis and influenza.

The term "E2 glycoprotein", as used herein, refers to a viral envelope glycoprotein having either the antigenic specificity or the biological function of the outer envelope glycoprotein 2 (E2) of HCV. The envelope glycoprotein E2 is an essential component of the HCV virion envelope and necessary for viral entry and fusion. E2 has a molecular weight of 70-72 kDa and assembles with the other HCV envelope glycoprotein E1 as noncovalent heterodimers. E1 and E2 are type I transmembrane glycoproteins located on the surface of viral lipid envelope, with N-terminal ectodomains of 160 and 334 residues, respectively, and a short C-terminal transmembrane domain of approximately 30 amino acids. The E1 and E2 transmembrane domains are composed of two stretches of hydrophobic amino acids separated by a short polar region containing conserved charged residues. E2 is highly modified post-translationally with 9-11 N-linked glycosylation sites and 18 cysteine residues that are conserved across all genotypes. Both E1 and E2 are essential for infectivity, with E2 possessing the binding sites for the entry receptors CD81 and SCARB1. The envelope glycoprotein E2 is the main target for neutralizing antibody responses, but is also the most variable antigen in HCV.

The term "guanine", as used herein, relates to one of the four main nucleobases found in nucleic acids DNA and RNA, the other being adenine (A), cytosine (C), and thymine (T) (DNA) or uracil (U) (RNA). Guanine is a derivative of purine, comprising a fused pyrimidine-imidazole ring system with conjugated double bonds. It binds to cytosine in genomic, double-stranded DNA, through three hydrogen bonds (Watson-Crick base pairing). Four guanine bases can also associate through Hoogsteen hydrogen bonding to form a square planar structure called a guanine tetrad (also known as G-tetrad or G-quartet). The term "guanosine" relates to a purine nucleoside comprising guanosine attached to a ribose (ribofuranose) ring via a β-N9-glycosidic bond. The term "deoxyguanosine" relates to a purine nucleoside comprising guanosine attached to the C1 carbon of a deoxyribose ring.

The term "G-quadruplex", also known as "quadruplex G", "G-tetrad", "G4-DNA", "tetraplex" or "G4 structure", relates to a secondary structure formed in a nucleic acid sequence that contains consecutive runs of guanine (G) bases forming G-quartets. A "G-quartet" (or Guanine quartet) is a planar association of four guanines bases linked through Hoogsteen hydrogen bonds. In a particular embodiment, the G-quadruplex structure is further stabilized by the presence of a monovalent cation, especially potassium, which locates in a central channel between each pair of G-quartets. G-quadruplexes can be formed from one, two or four separate strands of an oligonucleotide (DNA or RNA) according to the number of guanines and the number of guanines repetitions in the sequence of the oligonucleotide. A single sequence of at least four guanines induces the formation of four stranded tetrameric structures, termed tetramolecular G-quadruplex, where four oligonucleotides orientated in the same direction are linked through *n* G-quartets, with *n* the number of repeated guanines. Two series of repeated guanines form bi-molecular structures with two strands hold by G-quartets connected by loops. Four series of repeated guanines form mono (or uni-)molecular structures where one oligonucleotide fold four times upon itself when the guanines of the sequence form G-quartets. Mono- and bi-molecular species can display a wide variety of topologies, which are in part a consequence of various possible combinations of strand direction, as well as variations in loop size and sequence that connect the G-quartets. G-Quadruplexes are designated as anti-parallel when at least one of the four strands is anti-parallel to the others. Tetramolecular G-Quadruplexes contain parallel strands having all guanine glycosidic angles in an *anti* conformation. Mono- and bi-molecular G-Quadruplexes can have parallel and anti-parallel strands, with both *syn* and *anti* glycosidic angles for guanines, arranged in a way that is particular for a given topology and set of strand orientations. All G-quadruplex structures have four grooves, defined as the cavities bounded by the phosphodiester backbones.

G-quadruplexes according to the invention, also termed "G4-ON", can be formed of DNA, RNA, locked nucleic acid (LNA), and peptide nucleic acid (PNA). Similarly, G-quadruplexes according to the invention may be uni-molecular, bi-molecular or tetramolecular. Depending on the direction of the strands or parts of a strand that form the G-quartets, G-quadruplexes according to the invention may be parallel or antiparallel.

The term "gp120", as used herein, refers to a viral envelope glycoprotein having either the antigenic specificity or the biological function of the outer envelope protein (env) of HIV. A "gp120 protein" is a molecule derived from a gp120 region of an Env polypeptide. HIV envelope glycoprotein is synthesized as a precursor glycoprotein, gp160, and is then processed into gp120 and gp41. The mature gp120 polypeptides have about 511 amino acids in their primary sequence. gp120 is heavily N-glycosylated giving rise to an apparent molecular weight of 120kDa. gp120 contains five relatively conserved domains (C1-C5) interspersed with five variable domains (V1-V5). The variable domains contain extensive amino acid substitutions, insertions and deletions. The gp41 portion is anchored in (and spans) the membrane bilayer of the virion, while the gp120 segment protrudes into the surrounding environment. The receptor binding domain of gp120 is localized to N-terminal half of the protein. This is followed by a proline rich region (PRR), which is proposed to behave either as a hinge or trigger to communicate receptor binding to the fusion machinery. The C-terminus of the gp120 is highly conserved and interacts with the gp41. Exemplary sequences of wt gp160 polypeptides are available. See GenBank accession Nos. AAB05604 and AAD12142. Preferably, the gp120 polypeptide is derived from HIV Env.

The term "HIV", as used herein, includes HIV-1 and HIV-2 and SIV. "HIV- 1" means the human immunodeficiency virus type-1. HIV-1 includes, but is not limited to, extracellular virus particles and the forms of HIV-1 associated with HIV-1 infected cells. The HIV-1 virus may represent any of the known major groups M, N, O and P and the containing subtypes including laboratory strains and primary isolates. "HIV-2" means the human immunodeficiency virus type-2. HIV-2 includes, but is not limited to, extracellular virus particles and the forms of HIV-2 associated with HIV-2 infected cells. The term "SIV" refers to simian immunodeficiency virus which is an HIV-like virus that infects monkeys, chimpanzees, and other nonhuman primates. SIV includes, but is not limited to, extracellular virus particles and the forms of SIV associated with SIV infected cells. Preferably, HIV is HIV-1.

The expression "HIV infected subject", as used herein, refers to a human subject infected by HIV. HIV infected subjects are identified by clinical conditions associated with HIV infection and CD4+ T lymphocyte counts. From a practical standpoint, the clinician views HIV infection as a spectrum of disorders ranging from primary infection with or without the acute HIV syndrome to the symptomatic infection state of advanced disease, i.e. acquired immunodeficiency syndrome (AIDS). The Centers for Disease Control and Prevention (CDC) in Atlanta, Georgia, has established an authoritative definition for the diagnosis of AIDS, which informs a skilled artisan whether the onset of AIDS has occurred: in a HIV-infected subject, the CD4+ T cell count must be below 200 cells per cubic mm of blood, or there must be the clinical appearance of an initial AIDS-defining opportunistic infection, such as PCP (i.e. Pneumocystis carinii pneumonia), oral candidiasis (i.e. thrush), pulmonary tuberculosis, or invasive cervical carcinoma. Methods of determining a subject's CD4+ T cell count are known in the art.

The term "HCV", as used herein, includes HCV-1, HCV-2, HCV-3, HCV-4, HCV-5, HCV-6 and HCV-7 genotypes.

The expression "HCV infected subject", as used herein, refers to a human subject infected by HCV. The infection with HCV develops hepatitis C in the infected subject. There are a number of diagnostic tests for hepatitis C, including HCV antibody enzyme immunoassay or ELISA, recombinant immunoblot assay, and quantitative HCV RNA polymerase chain reaction (PCR).

The term "infection" or "microbial infection", as used herein, relates to invasion by bacteria, viruses, fungi, protozoa or other microorganisms, referring to the undesired proliferation or presence of invasion of pathogenic microbes in a host organism. It includes the excessive growth of microbes that are normally present in or on the body of a mammal or other organism. More generally, a microbial infection can be any situation in which the presence of a microbial population(s) is damaging to a host mammal. Thus, a microbial infection exists when excessive numbers of a microbial population are present in or on a mammal's body, or when the effects of the presence of a microbial population(s) is damaging the cells or other tissue of a mammal. In particular, in the context of the present invention, the infection is a viral infection, i.e. an infection caused by a virus. In a particular embodiment, the virus is an enveloped virus. In a more particular embodiment, the virus is selected from a virus of the *Retroviridae* family, a virus from the *Herpesviridae* family, and a virus from the *Flaviviridae* family. In a more particular embodiment, the retrovirus is selected from the group comprising human immunodeficiency virus 1 (HIV-1), human immunodeficiency virus 2 (HIV-2), human T-lymphotropic virus I (HTLV-I) and human T-lymphotropic virus II (HTLV-II). In a more particular embodiment, the retrovirus is selected from the group comprising HIV-1 and HIV-2, more preferably HIV-1. In a particular embodiment of the invention, the viral infection is a HIV infection. The term "HIV infection" relates to the verified presence of an HIV antibody, HIV antigen, or HIV nucleic acid in a subject as demonstrated by the detection of the presence of virus using HIV tests known to those skilled in the art (e.g. HIV EIA, Western blot, PCR tests). In a particular alternative embodiment, the virus is selected from a virus of the *Herpesviridae* family. In a more particular embodiment, the herpesvirus is selected from the group comprising cytomegalovirus (CMV), herpes simplex virus types 1 and 2 (HSV-1 and HSV-2), and Epstein-Barr virus (EBV). In a particular alternative embodiment, the virus is selected from a virus of the *Flaviviridae* family. In a more particular embodiment, the *Flaviviridae* virus is selected from the group comprising the Human Hepatitis C virus (HCV), Yellow fever virus, West Nile virus and Dengue virus.

The term "linker", as used herein, relates to a bi-functional unit that makes possible to link covalently the elements of the agent according to the invention, that is to say, to link an end of an oligonucleotide to a lipid moiety. In a particular embodiment, the linker links the 5' end of the polynucleotide of an agent according to the invention to a lipid moiety. In a particular alternative embodiment, the linker links the 3' end of the polynucleotide of an agent according to the invention to a lipid moiety. Suitable linkers according to the invention can be selected by the skilled person according to the particular lipid to be linked, as well as to the oligonucleotide end to be linked and include, without limitation, agents capable of linking the free hydroxyl group of an oligonucleotide, or the free phosphate group of an oligonucleotide. In a particular embodiment, the linker is selected from the group comprising glycerol, serinol, threoninol, hydroxyprolinol, any aminoalkyldiol, and derivatives thereof. In a particularly preferred embodiment, the linker is a derivative of glycerol, including without limitation, mono- and di-glycerides, and glycerol phosphates. In a particular embodiment, the derivative of glycerol is an unsaturated hydrocarbonated chain of C12-C20, including linoleyloxy group.

The term "lipophilic substituent moiety", as used herein, refers to a group of organic compounds that has lipophilic or amphipathic properties, including, but not limited to lipids, such as fats, fatty oils, essential oils, waxes, steroids, sterols, acylglycerides, phospholipids, phosphoglycerides, phosphosphingolipids, glucosphingolipids, terpenoids, glycolipids, sulpholipids, aminolipids, chromolipids (lipochromes), and fatty acids, as well as to alkyl substituents. The term "lipid" encompasses both naturally occurring and synthetically produced lipids. Suitable lipids that can be used include fatty acids; fats; oils; waxes; cholesterol; sterols; fat-soluble vitamins, such as vitamins A, D, E and K; monoglycerides; diglycerides, and phospholipids. Preferred fatty acids are those selected from the group consisting of octyl, decyl, dodecyl, tetradecyl, hexadecyl and octadecyl, octanoic acid, lauroic acid (C12), myristic acid (C14), palmitic acid (C16), stearic acid (C18), docosanoic acid (C22), and hybrid of lithocholic acid and oleylamine (lithocholic-oleyamine, C43). The term "alkyl substituent", as used herein, refers to an alkane of varying length missing one hydrogen. Preferred alkyls substituents are those containing an 8 to 30 carbon chain. The lipophilic substituent moiety may be selected without undue experimentation by the skilled person according to the circumstances by taking into consideration the target tissue, the target cell, the administration route, the pathway that the oligonucleotide is expected to follow, etc. Other lipophilic substituents may include cyclic alkanes, benzene and other aromatic compounds and heterocyclic compounds.

The term "oligonucleotide", as used herein, refers to a short, single-stranded DNA, RNA, LNA or PNA molecule, generally no greater than about 50 nucleotides. The oligonucleotides of the invention are preferably DNA molecules of at least 4, at least 5, at least 6, at least 7, at least 8, at least 9, at least 10, at least 11, at least 12, at least 13, at least 14, at least 15, at least 16, or at least 17 bases in length. It will be understood that when a nucleotide sequence is represented by a DNA sequence (i.e., A, T, G, C), this also includes an RNA sequence (i.e., A, U, G, C) in which "U" replaces "T". The term "LNA" or "locked nucleic acid", refers to a modified RNA nucleotide, wherein the ribose moiety is modified with an extra bridge connecting the 2' oxygen and 4' carbon. The bridge "locks" the ribose in the 3'-endo (North) conformation, which is often found in the A-form duplexes. The term "PNA" or "peptide nucleic acid", refers to an oligonucleotide having a backbone composed of repeating N-(2-aminoethyl)-glycine units linked by peptide bonds. LNA and PNA nucleotides can be mixed with DNA or RNA residues in the oligonucleotide whenever desired.

The term "pharmaceutical composition", as used herein, refers to a composition comprising a therapeutically effective amount of the agent according to the present invention and at least one pharmaceutically acceptable excipient. Pharmaceutical compositions according to the invention can be prepared, for instance, as injectables such as liquid solutions, suspensions, and emulsions.

The terms "pharmaceutically acceptable excipient", or "pharmaceutically acceptable carrier," "pharmaceutically acceptable diluent,", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could for example be selected from the group consisting of: AlK(SO₄)₂, AlNa(SO₄)₂, AlNH₄(SO₄)₂, silica, alum, Al(OH)₃, Ca₃(PO₄)₂, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1'2'-dipalmitoyl-sn-glycero-3-hydroxphosphoryloxy)-ethylamine (CGP 19835A, also referred to as MTP-PE), RIBI (MPL+TDM+CWS) in a 2% squalene/Tween-80®emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacteria spp, tuberculosis, substances found in Corynebacterium parvum, Bordetella pertussis, and members of the genus brucella, Titermax, ISCOMS, Quil A, ALUN, Lipid A derivatives, choleratoxin derivatives, HSP derivatives, LPS derivatives, synthetic peptide matrixes or GMDP, interleukin 1, interleukin 2, Montanide ISA-51 and QS-21, CpG oligonucleotide, poly I:C and GM-CSF. See Hunter R, US 5,554,372 and Jager E, et al., WO1997028816.

The term "polynucleotide", as used herein, refers to a polymer formed by a variable number of monomers wherein the monomers are nucleotides, including both ribonucleotides and deoxyribonucleotides. The terms "polynucleotide", oligonucleotide and "nucleic acid" are used interchangeably in this invention and include synthetic oligonucleotides. As used in this invention, the polynucleotides are not limited to polynucleotides as they appear in nature, but include polynucleotides containing non-natural nucleotide analogues and internucleotide bonds, and modified and unmodified polynucleotides.

The term "polypeptide" or "protein", as used herein, refers to a chain of amino acids of any length wherein the different amino acids are linked to one another by means of peptide bonds or disulphide bridges.

The terms "prevent," "preventing," and "prevention", as used herein, refer to a decrease in the occurrence of infection in a subject. The prevention may be complete (e.g. the total absence of infected cells in a subject). The prevention may also be partial, such that for example the occurrence of infected cells in a subject is less than that which would have occurred without the present invention. Prevention also refers to reduced susceptibility to a clinical condition or pathology.

The term "subject", as used herein, refers to an animal, in particular a vertebrate, such as a human, a non-human primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a particular embodiment, the subject is a human of any age or sex.

The term "therapeutically effective amount", as used herein in relation to the agent of the invention, or in relation to the agent, excipient and/or carrier comprised by the pharmaceutical composition of the invention, relates to the sufficient amount of said agent, excipient and/or carrier to provide the desired effect, i.e. to achieve an appreciable prevention, cure, delay, reduction of severity or amelioration of one or more symptoms derived from a viral infection, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated.

The term "therapy", as used herein, refers to the attempted remediation of a health problem, usually following a diagnosis, or to prevention or the appearance of a health problem. As such, it is not necessarily a cure, i.e. a complete reversion of a disease. This term includes both therapeutic treatment and prophylactic or preventative measures, in which the object is to prevent or stop (reduce) an undesired viral infection and/or a disease associated with an infection caused by an enveloped virus. For the purpose of this invention, beneficial or desired clinical results include, without limitation, relieving symptoms, reducing the spread of the disease, stabilising pathological state (specifically not worsening), slowing down or stopping the progression of the disease, improving or mitigating the pathological state and remission (both partial and complete), both detectable and undetectable. It can also involve prolonging survival in comparison with the expected survival if treatment is not received. Those subjects needing treatment include those subjects already diagnosed as being infected, as well as those with the tendency to be infected or those in which infection must be prevented.

The term "topical administration", as used herein, refers to the administration of an agent or a pharmaceutical composition on the skin or mucosae of a subject.

The term "treatment", as used anywhere herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a viral infection and/or a disease associated with an infection caused by an enveloped virus, as defined herein). Thus, "treatment," "treating," and the like, as used herein, refer to obtaining a desired pharmacologic and/or physiologic effect, covering any treatment of a viral infection and/or a disease associated with an infection caused by an enveloped virus in a mammal, including a human. The effect may be prophylactic in terms of completely or partially preventing a viral infection and/or may be therapeutic in terms of a partial or complete a disease associated with an infection caused by an enveloped virus. That is, "treatment" includes (1) preventing the viral infection from occurring or recurring in a subject, (2) inhibiting the viral infection, such as arresting its development, (3) stopping or terminating the viral infection or at least symptoms associated therewith, so that the host no longer suffers from the disorder or its symptoms, or (4) relieving, alleviating, or ameliorating the viral infection and/or a disease associated with an infection caused by an enveloped virus.

The term "virus", as used herein, refers to a small infectious agent that can replicate only inside the living cells of organisms. Non-limiting examples of viral families according to the present invention include *Adenoviridae,* African Swine Fever-Like Viruses, *Arenaviridae, Arterivirus, Astroviridae, Baculoviridae, Birnaviridae, Bunyaviridae*, *Caliciviridae, Circoviridae, Coronaviridae, Deltavirus, Filoviridae, Flaviviridae, Hepadnaviridae, Hepeviridae, Herpesviridae, Orthomyxoviridae, Paramyxoviridae, Picornaviridae, Poxyviridae, Reoviridae, Retroviridae,* and *Rhabdoviridae*. In a particular embodiment, the virus according to the invention is selected from the group comprising human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus (HSV), Epstein-Barr virus (EBV), respiratory syncytial virus (RSV), human cytomegalovirus (HCMV), human T-lymphotropic virus I (HLTV I) and human T-lymphotropic virus II (HLTV II). In a more particular embodiment, the virus according to the invention is a retrovirus. The term "retrovirus", as used herein, encompasses a class of viruses that belongs to the family *Retroviridae* and in which the genetic material is single-stranded RNA and which employ reverse transcriptase to transcribe the viral RNA into DNA in a host. Particularly intended herein are retroviruses infecting animals, more preferably retroviruses of warm-blooded animals, even more preferably of vertebrate animals, still more preferably of mammals, yet more preferably of primates, and most preferably of humans. In a particular embodiment, the retrovirus is selected from the group comprising human immunodeficiency virus 1 (HIV-1), human immunodeficiency virus 2 (HIV-2), human T-lymphotropic virus I (HTLV-I) and human T-lymphotropic virus II (HTLV-II). In a more particular embodiment, the retrovirus is selected from the group comprising HIV-1 and HIV-2, more preferably HIV-1. In an alternative particular embodiment of the invention, the virus is a herpesvirus. The term "herpesvirus", as used herein, relates to a virus of the *Herpesviridae* family, comprising enveloped, double-stranded DNA viruses with relatively large genomes. This family replicates in the nucleus of a wide range of vertebrate and invertebrate hosts, in preferred embodiments, mammalian hosts, for example in humans, horses, cattle, mice, and pigs. Exemplary members of the *Herpesviridae* family include cytomegalovirus (CMV), herpes simplex virus types 1 and 2 (HSV-1 and HSV-2), varicella zoster (VZV), Epstein-Barr virus (EBV) and HHV-8 (Kaposi's sarcoma herpesvirus). In a particular embodiment, the virus of the invention is an herpesvirus, preferably an herpesvirus selected from the group comprising cytomegalovirus (CMV), herpes simplex virus types 1 and 2 (HSV-1 and HSV-2), and Epstein-Barr virus (EBV). In a particular alternative embodiment, the virus is selected from a virus of the *Flaviviridae* family. The virus of the *Flaviviridae* family are single-stranded RNA viruses of positive polarity. Virus of this family are primarily spread through arthropod vectors (mainly ticks and mosquitoes) In a more particular embodiment, the *Flaviviridae* virus is selected from the group comprising human hepatitis C virus (HCV), Yellow fever virus, West Nile virus and Dengue Fever virus.

The term "viral envelope" or "envelope", as used herein, relates to the viral membrane, made of a lipid bilayer, covering the viral protein structure, termed Capsid, in which the viral genome is packaged. The viral envelope is derived from portions of the host cell membranes, especially issued from membrane microdomains related to rafts (phospholipids and cholesterol). The viral envelope includes viral envelope glycoproteins that serve to identify and bind to receptors and co-receptors on the host's cell membrane, which promotes the viral entry into its target cell.

The term "viral envelope glycoprotein", as used herein, relates to viral glycoproteins anchored in the viral membrane.

### 1. Antiviral agent of the invention

The authors of the invention have developed non-cytotoxic agents based on a DNA-lipid conjugate forming a G-quadruplex structure that have potent antiviral activity against enveloped virus, such as HIV and HCV. These agents display high stability and submicromolar efficiency as viral entry inhibitors of both HIV and HCV strains.

Thus, in a first aspect, the invention relates to an agent comprising one unit, two units or four units, wherein each of said units comprises:
(i) an oligonucleotide comprising at least four consecutive guanines (G), wherein said at least four consecutive G form an intra-unit (intra-molecular) or an inter-unit (inter-molecular) G-quadruplex, wherein said G-quadruplex binds to a viral envelope glycoprotein, which inhibits the binding of said viral envelope glycoprotein to a cellular receptor and/or co-receptor of said viral envelope glycoprotein,
and wherein at least one of said units comprises:
(ii) a lipophilic substituent moiety, wherein said lipophilic molecule anchors to a viral envelope or to the membrane of a cell susceptible of being infected by a virus, and
(iii) a linker, wherein said linker links the 5' or 3' end of the oligonucleotide in (i) to the lipophilic substituent moiety in (ii),
wherein said agent has antiviral activity.

The agent according to the invention comprises one unit, two units, or four units, wherein each of said units comprises an oligonucleotide, and wherein at least one of said units further comprises a lipophilic substituent moiety, and a linker, and wherein said agent has antiviral activity.

### A. Oligonucleotide of the agent of the invention

The oligonucleotide comprised by the agent of the invention comprises at least four contiguous, consecutive guanines (G), wherein said at least four consecutive, contiguous G form an intra-unit (intra-molecular) or an inter-unit (intra-molecular) G-quadruplex. According to the invention, the G-quadruplex comprising the oligonucleotide of the agent of the invention binds to a viral envelope glycoprotein.

Therefore, the G-quadruplex comprising the oligonucleotide of the agent of the invention interferes with the contact between a virus and a cell susceptible of being infected by said virus, by binding to a viral envelope glycoprotein, which inhibits the binding of the said viral envelope glycoprotein to a receptor or/and a co-receptor of said viral envelope glycoprotein at the surface of a cell susceptible of being infected by said virus.

Assays to determine whether a G-quadruplex binds to a viral envelope glycoprotein are known in the art and include, without limitation, electrophoretic mobility shift assay (EMSA), Surface Plasmon Resonance (SPR), Pull-down assay, gel filtration assay, ligand/EnvGP binding assay, ELISA ligand/EnvGP assay, molecular dynamics simulation, as well as the methods disclosed in WO 2005037997 A2.

Assays to determine whether a molecule, such as a G-quadruplex oligonucleotide, interferes the contact between a virus and a cell susceptible of being infected by said virus by binding to a viral envelope glycoprotein are known by the skilled person, and include cell-to-cell virus transfer assay, time of addition assay, cell-based fusion assay. Particular examples of these assays are described in the Materials and Methods section of the Examples of the present specification (Subsections "3. Evaluation of anti-HIV activity" and "4. Evaluation of anti-HCV activity").

In a particular embodiment, said enveloped virus is selected from the group comprising human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus, Epstein Barr virus, respiratory syncytial virus, human cytomegalovirus, human T-lymphotropic virus I (HLTV I) and human T-lymphotropic virus II (HLTV II).

In a preferred embodiment, the enveloped virus is HIV, preferably HIV-1. Thus, and the viral envelope glycoprotein is an envelope glycoprotein from HIV-1. In a particularly preferred embodiment, the HIV-1 viral EnvGP is gp120.

In another preferred embodiment, the enveloped virus is HCV. Thus, the viral envelope glycoprotein is an envelope glycoprotein from HCV. In a particularly preferred embodiment, the HCV viral EnvGP is a glycoprotein selected from the group consisting of EnvGP E1 and EnvGP E2.

In an alternative or additional embodiment, the G-quadruplex oligonucleotide comprised by the agent of the invention binds to a viral envelope glycoprotein. In a particular embodiment, the viral envelope glycoprotein is a protein from an enveloped-virus. In a more particular embodiment, the enveloped virus is selected from the group comprising viruses from the *Retroviridae* family and viruses from the *Herpesviridae* family. In a more particular embodiment, the viral envelope glycoprotein is a protein from a virus from the *Retroviridae* family selected from the group comprising human immunodeficiency virus 1 (HIV-1), human immunodeficiency virus 2 (HIV-2), human T-lymphotropic virus I (HTLV-I) and human T-lymphotropic virus II (HTLV-II). In a particular preferred embodiment, the viral envelope glycoprotein is an envelope protein from HIV-I. In a particular preferred embodiment, the HIV-1 envelope glycoprotein is gp120. In an alternative particular embodiment, the viral envelope protein is a protein from a virus from the *Herpesviridae* family selected from the group comprising cytomegalovirus (CMV), herpes simplex virus types 1 and 2 (HSV-1 and HSV-2), and Epstein-Barr virus (EBV). In another particular alternative embodiment, the virus is selected from a virus of the *Flaviviridae* family. In a more particular embodiment, the *Flaviviridae* virus is selected from the group comprising HCV, Yellow fever virus, West Nile virus and Dengue virus. In a preferred embodiment, the virus is HCV and the viral envelope protein is selected from protein E1 and protein E2.

Methods to determine whether an oligonucleotide sequence can form an intramolecular or an intermolecular G-quadruplex are described in the art. Parameters to be taken into consideration when determining if an oligonucleotide may or may not arrange in an intramolecular or an intermolecular G-quadruplex include the number of G residues in the sequence of the oligonucleotide, the number of consecutive G residues in the sequence of the oligonucleotide, the length of the sequence, and the number of nucleotides located between the repeated G. In this regard, bioinformatics programs are available for prediction and/or analysis of G-quadruplexes in a nucleotide sequence including, without limitation, the QGRS Mapper from Ramapo College Bioinformatics (available at http://bioinformatics.ramapo.edu/QGRS/analyze.php, for analysis of monomolecular folding of G-rich sequences, see Menendez C et al. 2012 Nucleic Acids Res 40: W96-W103), the predictor for G-quadruplex stability (available at http://www.inference.phy.cam.ac.uk/os252/projects/quadruplexes/ and Stegle O et al. 2009 Bioinformatics 25(12):i374-382). A database of prokaryotic (ProQuad) and Eukaryotic (EuQuad) quadruplex sequences is available at http://quadbase.igib.res.in/. Methods for prediction of G-quadruplex formation and stability are available in the art including, without limitation, those described in Wong HM et al. 2010 J Nucleic Acids (doi: 10.4061/2010/564946).

As previously mentioned, the oligonucleotide comprised by the agent of the invention comprises at least four consecutive contiguous G, wherein said at least four consecutive G form an intra-unit (intra-molecular) or an inter-unit (inter-molecular) G-quadruplex. In another particular embodiment, the oligonucleotide comprises the nucleotide sequence

5'- X₁ X₂ G G G (G)ᵢ X₃ (SEQ ID NO: 8) (tail) - 3'

wherein
- X₁ may be present or absent, and represents any nucleotide,
- X₂ represents T, A or C, preferably T or A,
- i is an integer between 1 and 4,
- X₃ represents T, A or C, preferably T or A,
- (tail) represents a nucleotide sequence, comprising between 0 and 23 nucleotides.

In a preferred embodiment, i is 3.

### A.1. Agent of the invention comprising a monomolecular G-quadruplex,

In a particular embodiment, the agent according to the invention comprises one unit, wherein said unit comprises an oligonucleotide, a lipophilic substituent moiety and a linker according to the invention. When the agent of the invention comprises one unit as previously described, the oligonucleotide portion of said unit is arranged in a monomolecular G-quadruplex. A monomolecular G-quadruplex arrangement is the result of an intra-unit (intra-molecular) G-quadruplex, wherein the oligonucleotide doubles on itself and is maintained by intramolecular Hoogsteen bonds established between G nucleotides located in the oligonucleotide portion of the unit of the agent of the invention.

Any oligonucleotide sequence comprising the nucleotide sequence 5'- X₁ X₂ G G G (G)ᵢ X₃ (SEQ ID NO:8) (tail) - 3', wherein said oligonucleotide is arranged in a monomolecular G-quadruplex, may be comprised by the anti-viral agent according to the present invention. Methods to determine whether a nucleotide sequence may arrange in a monomolecular G-quadruplex are known by the skilled person and have been described previously.

Therefore, when the agent of the invention comprises one unit, wherein said unit comprises an oligonucleotide, a lipophilic substituent moiety and a linker according to the invention, the oligonucleotide is arranged in an intramolecular G-quadruplex. In a particular embodiment, said oligonucleotide comprises the nucleotide sequence:

5'- X₁ X₂ G G G (G)ᵢ X₃ (SEQ ID NO:8) (tail) - 3'

wherein
- X₁ may be present or absent, and represents any nucleotide,
- X₂ represents T, A or C, preferably T or A,
- i is an integer between 1 and 4,
- X₃ represents T, A or C, preferably T or A,
- (tail) represents a nucleotide sequence, comprising between 1 and 23 nucleotides.

In a particular embodiment, the oligonucleotide arranged in an intramolecular G-quadruplex of the agent of the invention comprises a tail, wherein said tail comprises three repetitions of at least three consecutive G, wherein each of said at least three consecutive G is separated from the following at least three consecutive G by at least one nucleotide.

In a more particular embodiment, the oligonucleotide arranged in an intramolecular G-quadruplex of the agent of the invention comprises the sequence 5'-TTGGGGATAGGGATAGGGATAGGGATA-3' (SEQ ID NO: 1). Preferably, the oligonucleotide consists of the sequence SEQ ID NO: 1.

### A.2. Agent of the invention comprising a bimolecular G-quadruplex,

In a particular alternative embodiment, the agent according to the invention comprises two units, wherein each of said units comprises an oligonucleotide, a lipophilic substituent moiety and a linker according to the invention. When the agent of the invention comprises two units as previously described, the oligonucleotide portion of said two units is arranged in a bimolecular G-quadruplex. A bimolecular G-quadruplex arrangement is the result of an inter-unit (inter-molecular) interaction, wherein the Hoogsteen interactions are established between G nucleotides located in the oligonucleotide portion of the two units of the agent of the invention. The bimolecular G-quadruplex arrangement may be parallel or anti-parallel. In the parallel disposition, both oligonucleotides are arranged in 5'3' direction (or both are arranged in 3'5' direction). In the anti-parallel disposition, the first oligonucleotide is arranged in 5'3' direction, and the second oligonucleotide is arranged in 3'5' direction, wherein the terms first and second are arbitrary and may designate any of the two oligonucleotides.

In a particular embodiment, the lipophilic substituent moiety comprised by the first unit of the bimolecular G-quadruplex is different from lipophilic substituent moiety comprised by the second unit of the bimolecular G-quadruplex, wherein first and second unit are used interchangeably to designate any of the two units of the agent of the invention. In an alternative preferred embodiment, the lipophilic substituent moiety comprised by the first unit of the bimolecular G-quadruplex is the same as the lipophilic substituent moiety comprised by the second unit of the bimolecular G-quadruplex, wherein first and second unit are used interchangeably to designate any of the two units of the agent of the invention.

In an alternative embodiment, the present invention also contemplates that one of the two units of the agent of the invention comprising a bi-molecular G-quadruplex comprises a lipophilic substituent moiety, wherein said lipophilic molecule anchors to a viral envelope or to the membrane of a cell susceptible of being infected by a virus, and a linker, wherein said linker links the 5' or 3' end of the oligonucleotide in (i) to the lipophilic substituent moiety.

Any oligonucleotide sequence comprising the nucleotide sequence 5'- X₁ X₂ G G G (G)ᵢ X₃ (SEQ ID NO:8) (tail) - 3', wherein said oligonucleotide is arranged in a bimolecular G-quadruplex, may be comprised by the anti-viral agent according to the present invention. Methods to determine whether a nucleotide sequence may arrange in a bimolecular G-quadruplex are known by the skilled person and have been described previously.

In a particular embodiment, the oligonucleotides comprised by the two units of the agent of the invention and arranged in an intermolecular, bimolecular G-quadruplex comprise the nucleotide sequence:

5'- X₁ X₂ G G G (G)ᵢ X₃ (SEQ ID NO:8) (tail) - 3'

wherein
- X₁ may be present or absent, and represents any nucleotide,
- X₂ represents T, A or C, preferably T or A,
- i is an integer between 1 and 4,
- X₃ represents T, A or C, preferably T or A,
- (tail) represents a nucleotide sequence, comprising between 1 and 23 nucleotides.

In a particular embodiment, the oligonucleotides arranged in an intermolecular, bimolecular G-quadruplex of the agent of the invention comprise a tail, wherein said tail comprises at least three consecutive G.

### A.3. Agent of the invention comprising a tetramolecular G-quadruplex,

In a particular preferred embodiment, the agent according to the invention comprises four units, wherein each of said units comprises an oligonucleotide, a lipophilic substituent moiety and a linker according to the invention. When the agent of the invention comprises four units as previously described, the oligonucleotide portion of said four units is arranged in an intermolecular, tetramolecular G-quadruplex. A tetramolecular G-quadruplex arrangement is the result of an inter-unit (inter-molecular) interaction, wherein the Hoogsteen interactions are established between G nucleotides located in the oligonucleotide portion of the four units of the agent of the invention. The tetramolecular G-quadruplex arrangement may be parallel or anti-parallel. In the parallel disposition, all oligonucleotides are arranged in 5'3' direction (or all of them are arranged in 3'5' direction).

Thus, in a particular preferred embodiment of the anti-viral agent of the invention, said agent comprises four units, wherein each of said units comprises an oligonucleotide, a lipophilic substituent moiety and a linker according to the invention, and wherein said oligonucleotides are arranged in a tetramolecular parallel G-quadruplex.

Any oligonucleotide sequence comprising the nucleotide sequence 5'- X₁ X₂ G G G (G)ᵢ X₃ (SEQ ID NO:8) (tail) - 3', wherein said oligonucleotide is arranged in a tetramolecular G-quadruplex, may be comprised by the anti-viral agent according to the present invention. Methods to determine whether a nucleotide sequence may arrange in a tetramolecular G-quadruplex are known by the skilled person and have been described previously.

In a particular embodiment, the lipophilic substituent moiety comprised by the first unit of the bimolecular G-quadruplex is different from lipophilic substituent moiety comprised by the second, third, and/or fourth unit(s) of the tetramolecular G-quadruplex, wherein said first, second, third and fourth units are used interchangeably to designate any of the four units of the agent of the invention. In an alternative preferred embodiment, the lipophilic substituent moiety comprised by the first unit of the tetramolecular G-quadruplex is the same as the lipophilic substituent moiety comprised by the second, third, and fourth units of the tetramolecular G-quadruplex, wherein said first, second, third and fourth units are used interchangeably to designate any of the four units of the agent of the invention. In an alternative embodiment, the present invention also contemplates that at least one, at least two, or at least three of the units of the agent of the invention comprise a lipophilic substituent moiety, wherein said lipophilic molecule anchors to a viral envelope or to the membrane of a cell susceptible of being infected by a virus, and a linker, wherein said linker links the 5' or 3' end of the oligonucleotide in (i) to the lipophilic substituent moiety.

Therefore, in a particular preferred embodiment, the agent of the invention comprises four units, wherein each of said units comprises an oligonucleotide, a lipophilic substituent moiety and a linker according to the invention, and wherein the oligonucleotides are arranged in a tetramolecular G-quadruplex, preferably a tetramolecular parallel G-quadruplex. Each of the oligonucleotides comprised by the four units of the agent of the invention comprises the nucleotide sequence

5'- X₁ X₂ G G G (G)ᵢ X₃ (SEQ ID NO:8) (tail) - 3'

wherein
- X₁ may be present or absent, and represents any nucleotide,
- X₂ represents T, A or C, preferably T or A,
- i is an integer between 1 and 4,
- X₃ represents T, A or C, preferably T or A,
- (tail) represents a nucleotide sequence, comprising between 1 and 23 nucleotides.

In a particular embodiment, the oligonucleotides arranged in an intermolecular, tetramolecular G-quadruplex of the agent of the invention comprise a tail, wherein said tail does not comprise at least two consecutive contiguous G.

In a more particular embodiment, the oligonucleotides arranged in an intermolecular, tetramolecular G-quadruplex of the agent of the invention comprise a tail, wherein said tail does not comprise at least three consecutive contiguous G.

In a preferred embodiment, the oligonucleotides arranged in an intermolecular, tetramolecular G-quadruplex of the agent of the invention comprise the sequence 5'-TTGGGGGGTAC-3' (SEQ ID NO: 2). Preferably, the oligonucleotide consists of the sequence SEQ ID NO: 2. In a more preferred embodiment, the oligonucleotides arranged in an intermolecular, tetramolecular G-quadruplex of the agent of the invention comprise the sequence 5'-TTGGGGGGTACAGTGCA-3' (SEQ ID NO: 3). Preferably, the oligonucleotide consists of the sequence SEQ ID NO: 3.

The backbone which connects the nucleotides of the oligonucleotide comprised by the one, two of four units of the agent of the invention can be a phosphodiester link. In an alternative embodiment, the backbone which connects the nucleotides of the oligonucleotide comprised by the one, two of four units of the agent of the invention can be a phosphorothioate linkage.

The oligonucleotide comprised by the agent of the invention may contain one or more modifications in the nucleobases, in the sugars and/or in the internucleotide linkages.

Modifications to one or more backbone residues of the nucleic acids may comprise one or more of the following: 2' sugar modifications such as 2'-O-methyl (2'-OMe), 2'-O-methoxyethyl (2'-MOE), 2'-O-methoxyethoxy, 2'- Fluoro (2'-F), 2'-AllylI, 2'-O-[2-(methylamino)-2-oxoethyl], 2'-O-(N-methylcarbamate); 4' sugar modifications including 4'-thio, 4'-CH₂-O-2'-bridge, 4-(CH₂)₂-O-2'-bridge; Locked Nucleic Acid (LNA); Peptide Nucleic Acid (PNA); Intercalating nucleic acid (INA); Twisted intercalating nucleic acid (TINA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers (Que-Gewirth NS, Gene Ther. 2007 Feb;14(4):283-91.); RNA Aptamers regulated with antidotes on the subject of the specific RNA aptamer (ref. Oney S, Oligonucleotides. 2007 Fall;17(3):265-74.), or any combinations thereof.

Modifications to one or more internucleoside linkages of the nucleic acids may comprise one or more of the following: phosphorothioate, phosphoramidate, phosphorodiamidate, phosphorodithioate, phosphoroselenoate, phosphorodiselenoate, phosphoroanilothioate and phosphoranilidate, or any combinations thereof.

Intercalating nucleic acid (INA) is a modified nucleic acid analogue comprised of normal deoxyribonucleotides covalently linked to hydrophobic insertions. INA has high affinity for complementary DNA with stabilization of up to 11 degrees for each modification. INA has a higher specificity for a fully matched target over mismatched targets than normal DNA. Utilizing that INAs have higher affinity for DNA makes it possible to use shorter probes and thereby enhance specificity even further. Further, INA is a DNA selective oligonucleotide analogue, with a unique ability to discriminate between DNA and RNA. Even though INAs have high affinities for complementary DNA, it has a lower affinity for a complementary sequence of complementary INAs. Twisted intercalating nucleic acids are denoted TINA.

Hexitol nucleic acids (HNA) are oligonucleotides built up from natural nucleobases and a phosphorylated 1,5-anhydrohexitol backbone. Molecular associations between HNA and RNA are more stable than between HNA and DNA and between natural nucleic acids (dsDNA, dsRNA, DNA/RNA). Other synthetically modified oligonucleotides comprise ANA (arabinonucleic acid), CNA (cyclohexane nucleic acids), CeNA (cyclohexenylnucleic acid) and TNA (threosyl nucleic acid).

Morpholinos are synthetic molecules which are the product of a redesign of the natural nucleic acid structure. Structurally, the difference between morpholinos and DNA or RNA is that while Morpholinos have standard nucleobases, those bases are bound to 6-membered morpholine rings instead of deoxyribose/ribose rings and nonionic phosphorodiamidate intersubunit linkages replace anionic phosphodiester linkages. Morpholinos are sometimes referred to as PMO (phosphorodiamidate morpholino oligonucleotide). The 6-membered morpholine ring has the chemical formula O-(CH₂-CH₂)₂-NH.

Gapmers are RNA-DNA-RNA chimeric oligonucleotide probes, where windows or 'gaps' of DNA are inserted into an otherwise normal or modified RNA oligonucleotide. This modification increases oligonucleotide stability in vivo and the avidity of the interaction of the probe with the target, so that shorter probes can be used effectively.

### B. Lipid of the agent of the invention

A second element of the agent of the invention is a lipophilic substituent moiety, wherein said lipophilic substituent moiety tethers the G-quadruplex to a viral envelope or to the membrane of a cell susceptible of being infected by a virus. Thus, the lipophilic substituent moiety comprised by at least one of the units of the agent of the invention allows said agent to be tethered or anchored to the viral envelope of a virus or to the membrane of a cell which is susceptible of being infected by said virus.

In a particular embodiment, the anti-viral agent of the invention comprises one unit, wherein said unit comprises (i) an oligonucleotide comprising at least four consecutive guanines (G), wherein said at least four consecutive G form an intra-unit (intra-molecular) G-quadruplex, wherein said G-quadruplex binds to a viral envelope glycoprotein, (ii) a lipophilic substituent moiety, wherein said lipophilic molecule anchors to a viral envelope or to the membrane of a cell susceptible of being infected by a virus, and (iii) a linker, wherein said linker links the 5' or 3' end of the oligonucleotide in (i) to the lipophilic substituent moiety in (ii).

In a particular alternative embodiment, the anti-viral agent of the invention comprises two units, wherein each of said units comprises (i) an oligonucleotide comprising at least four consecutive guanines (G), wherein said at least four consecutive G form an inter-unit (inter-molecular) bimolecular G-quadruplex, wherein said G-quadruplex binds to a viral envelope glycoprotein, and wherein at least one of said units comprises (ii) a lipophilic substituent moiety, wherein said lipophilic molecule anchors to a viral envelope or to the membrane of a cell susceptible of being infected by a virus, and (iii) a linker, wherein said linker links the 5' or 3' end of the oligonucleotide in (i) to the lipophilic substituent moiety in (ii). In a preferred embodiment, the anti-viral agent of the invention comprises two units, wherein each of said units comprises (i) an oligonucleotide, (ii) a lipophilic substituent moiety and (iii) a linker as above.

The lipophilic substituent moiety comprised by the first unit of the bimolecular G-quadruplex is different from lipophilic substituent moiety comprised by the second unit of the bimolecular G-quadruplex, wherein first and second unit are used interchangeably to designate any of the two units of the agent of the invention. In an alternative preferred embodiment, the lipophilic substituent moiety comprised by the first unit of the bimolecular G-quadruplex is the same as the lipophilic substituent moiety comprised by the second unit of the bimolecular G-quadruplex, wherein first and second unit are used interchangeably to designate any of the two units of the agent of the invention.

In another alternative embodiment, the anti-viral agent of the invention comprises four units, wherein each of said units comprises (i) an oligonucleotide comprising at least four consecutive guanines (G), wherein said at least four consecutive G form an inter-unit (inter-molecular) tetramolecular G-quadruplex, wherein said G-quadruplex binds to a viral envelope glycoprotein and wherein at least one of said units comprises (ii) a lipophilic substituent moiety, wherein said lipophilic molecule anchors to a viral envelope or to the membrane of a cell susceptible of being infected by a virus, and (iii) a linker, wherein said linker links the 5' or 3' end of the oligonucleotide in (i) to the lipophilic substituent moiety in (ii). In a preferred embodiment, the anti-viral agent of the invention comprises four units, wherein each of said units comprises (i) an oligonucleotide, (ii) a lipophilic substituent moiety and (iii) a linker as above.

In a particular embodiment, the lipophilic substituent moiety comprised by the first unit of the bimolecular G-quadruplex is different from lipophilic substituent moiety comprised by the second, third, and/or fourth unit(s) of the tetramolecular G-quadruplex, wherein said first, second, third and fourth units are used interchangeably to designate any of the four units of the agent of the invention. In an alternative preferred embodiment, the lipophilic substituent moiety comprised by the first unit of the tetramolecular G-quadruplex is the same as the lipophilic substituent moiety comprised by the second, third, and fourth units of the tetramolecular G-quadruplex, wherein said first, second, third and fourth units are used interchangeably to designate any of the four units of the agent of the invention.

In a particular embodiment, the lipophilic molecule or lipophilic substituent moiety of the agent according to the invention is a lipid selected from the group consisting of a fatty acid, and acylglyceride, a phospholipid, a glucolipid, a terpenoid and a steroid.

The term "fatty acid", as used herein, refers to long chain aliphatic acids (alkanoic acids) of varying chain lengths, from C₈ to C₃₀, preferably from C₁₂ to C₂₂, containing none, one or more than one insaturation. Preferably, the fatty acid is selected from the group of stearic acid (18:0 or octadecanoic acid), oleic acid (18:1 cis-9 or (9*Z*)-octadec-9-enoic acid), palmitic acid (16:0 or hexadecanoic acid), linoleic acid (18:2(ω-6) or *cis, cis*-9,12-octadecadienoic acid), arachidonic acid (20:4 (ω-6) or *all-cis-*5,8,11,14-eicosatetraenoic acid), docosohexanoic acid (22:6 (n-3 or (4*Z*,7*Z*,10Z,13*Z*,16*Z*,19*Z*)-docosa-4,7,10,13,16,19-hexaenoic acid). In a particular embodiment, the fatty acid is stearic, oleic, linoleic, linolenic, linoelainic, miristoleic, palmitoleic, sapienic, elaidic, vaccenic acids and their corresponding alcohol derivatives.

The term "acylglyceride" or "acyl gliceride", as used herein, relates to a glyceride consisting of one, two, or three fatty acid chains covalently bonded to a glycerol molecule through ester linkages. Acyl glycerides include monoglycerides (monoacylglicerols), diglycerides (also known as diacylglycerol or DAG) and triglycerides (also known as triacylglycerol, TG, triacylglyceride, or TAG) comprising, respectively, one, two or three fatty acid chains. Monoglycerides include 1-monoacylglycerols and 2-monoacylglycerols, depending on the position of the ester bond on the glycerol moiety. Exemplary monoglycerides include 2-arachidonoylglycerol and the glycerides of all the above mentioned fatty acids. Diglycerides comprise, without limitation, 1-palmitoyl-2-oleoyl-glycerol.

The term "phospholipid", as used herein, refers to a lipid that contains one or more phosphate groups. Phospholipids are amphipathic in nature; that is, each molecule consists of a hydrophilic (water-loving) portion and a hydrophobic (water-hating) portion. Phospholipids can be classified according to the type of alcohol in "phosphoglycerides" (or glycerophospholipids), when they carry a glycerol backbone, and "phosphosphingolipids" wherein the lipids contain sphingosine. Both classes are present in the biological membrane. Phosphoglycerides are the most abundant class of phospholipids found in nature and include, without limitation, phosphatidylcholine (lecithin), phosphatidylethanolamine, phosphatidylserine, phosphatidylinositol, phosphatidylglycerol, and cardiolipin. The structural diversity within each type of phosphoglyceride is due to the variability of the chain length and degree of saturation of the fatty acid ester groups. Sphingomyelin is the major sphingosine-containing phospholipid. Its general structure consists of a fatty acid attached to sphingosine by an amide linkage.

The term "glucolipid" or "glycolipid", as used herein, relates to a lipid including a residue of carbohydrate. Glycolipids comprise glyceroglycolipids (including galactolipids and sulfolipids), glycosphingolipids (including cerebrosides - such as galactocerebrosides, glucocerebrosides and sulfatides-, gangliosides, globosides, and glycophosphosphingolipids) and glycosylphosphatidylinositols. According to the type of a lipid portion, glycolipid is classified into glycosphingolipid, glycoglycerolipid, and other glycolipid, and examples of these glycolipids can include, but are not limited to, ganglioside GM3 and ganglioside GM4. Typical glycosphingolipid includes neutral glycosphingolipids such as galactocerebroside glucocerebroside, and globoside, and acidic glycosphingolipids such as ganglioside. Glycoglycerolipid can include monogalactosyldiacylglycerol, digalactosyldiacylglycerol, and the like. Other kinds of glycolipids, such as glycolipid containing uronic acid (uronic acid-containing glycolipid), phosphonoglycolipid containing phosphonic acid, and phosphoglycolipid containing phosphoric acid have been found. Preferably, the glucolipid is selected from the group comprising glycosphingolipids, glyceroglycolipids, rhamnolipids.

The term "terpenoid", also known as isoprenoid, relates to a large and diverse class of naturally occurring organic chemicals similar to terpenes, derived from five-carbon isoprene units assembled and modified in a large number of ways. Most are multicyclic structures that differ from one another not only in functional groups but also in their basic carbon skeletons. As used herein, this term is intended to cover terpenes and derivatives thereof having at least one C₅H₈ hydrocarbon unit with one or more points of unsaturation within the chemical structure, including monoterpenes, sesquiterpenes, diterpenes, sesteterpenes and triterpenes. Preferably, the terpenoid is selected from the group comprising sterols, lanosterol, bisabolol, squalene, retinol, phytol, taxol.

The term "steroid", as used herein, relates to an organic compound comprising a characteristic arrangement of four cycloalkane rings that are joined to each other, composed of twenty carbon atoms bonded together that take the form of four fused rings: three cyclohexane rings and one cyclopentane ring. Steroids include, without limitation, cholesterol, ergoesterol, stigmasterol, ergocalciferol, precalciferol, ecdysone, cholic acid, pregnenolone, progesterone, cortisone and testosterone. Preferably, the steroid is selected from the group comprising cholesterol, cholic acid, estradiol, testosterone, cortisol, progesterone, dexamethasone.

In another particular embodiment of the invention, the lipophilic substituent moiety is an alkyl substituent, preferably a C8-C30 alkyl substituent, more preferably a C₁₂ to C₂₂ alkyl substituent.

In a preferred embodiment, the lipophilic substituent moiety is stearic acid. In another preferred embodiment, the lipophilic substituent moiety is cholesterol.

The person skilled in the art will appreciate that it may be advantageous to have the lipophilic substituent moiety attached to the 5' end and/or to the 3' end, depending on the particular circumstances of the target virus and the oligonucleotide of the agent of the invention. Thus, in a particular embodiment, the lipophilic substituent moiety is attached to the 5' end of the oligonucleotide of the agent of the invention. In another particular embodiment, the lipophilic substituent moiety is attached to the 3' end of the oligonucleotide of the agent of the invention. In another particular embodiment, the lipophilic substituent moiety is attached to both the 5' and the 3' ends of the oligonucleotide of the agent of the invention.

### C. Linker of the agent of the invention

As described previously, at least one of the units of the anti-viral agent of the invention comprises (i) an oligonucleotide, (ii) a lipophilic substituent moiety, and (iii) a linker, wherein said linker links the 5' or 3' end of the oligonucleotide in (i) to the lipophilic substituent moiety in (ii).

In a particular embodiment, the linker is selected from the group comprising derivatives of glycerol (comprising, without limitation, glyceraldehyde, glyceric acid, hihydroxy acetone, hydroxy-piruvic aldehyde, hydroxyl-pyroracemic acid, tartronic semi-aldehyde, tartronic acid, tartronic dialdehyde, mesoxalic dialdehyde, mesoxalic semi-aldehyde and mesoxalic acid), serinol, threoninol, hydroxyprolinol, or any aminoalkyldiol such as threoninol or alkyltriol such as glycerol.

### D. Additional elements of the agent of the invention

The oligonucleotide(s) comprised by the anti-viral agent of the invention have to be preserved from degrading factors, such as nucleases (endo/exonucleases). With this aim, the oligonucleotides are designed to resist the enzymatic digestion, and to improve the *in vivo* stability and bioavailability of the oligonucleotide comprised by the anti-viral agent. Preferably, oligonucleotide(s) of the agent of the invention are chemically modified by the presence of a group which prevents nuclease-mediated degradation.

Therefore, in a particular embodiment, the one, two or four units comprised by the agent of the invention further comprise(s) a protecting group. For each unit, the protecting group is bound to the end of the oligonucleotide which is not bound to the linker. Therefore, if the 5' end of the oligonucleotide is bound to the linker, the protecting group is bound to the 3' end of the oligonucleotide. Alternatively, if the 3' end of the oligonucleotide is bound to the linker, the protecting group is bound to the 5' end of the oligonucleotide. In a preferred embodiment, the protecting group is bound to the 5' end of the oligonucleotide via phosphate, amide, ether, triazol, or ester groups.

Suitable protecting groups are well known for the skilled person in the art. Representative amino protecting groups include poly-L-lysine, propylamine formyl, acetyl, trifluoroacetyl, benzyl, benzyloxycarbonyl (CBZ), tert-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitroveratryloxycarbonyl (NVOC) and the like. Representative hydroxy protecting groups include those where the hydroxy group is either acylated or alkylated such as benzyl and trityl ethers as well as alkyl ethers, tetrahydropyranyl ethers, trialkylsilyl ethers and allyl ethers, and also cyanoethyl groups. Additionally, hydroxy groups can be protected by photoremovable groups such as a-methyl-6-nitropiperonyloxycarbonyl. Examples of protected phosphate groups include, without limitation, 2-cyanoethyl phosphoramidites.

In a particular embodiment, the protecting group is selected from the group consisting of poly-L-lysine and propylamine.

Localization of the anti-viral agent of the invention can be of interest. Therefore, in another particular embodiment, the agent of the invention further comprises a detectable tag, wherein said detectable tag can be detected either directly due to any property of said compound or indirectly because said compound has the capacity to modify a second molecule which is detectable. In a particular embodiment, the detectable tag is selected from the group comprising biotin, a fluorescent dye, a luminescent group or an enzyme.

In a particular embodiment, the detectable compound is an enzyme, wherein said enzyme is capable of generating a detectable signal, for example, after adding an activator, substrate, amplifying agent and the like. The enzymes which are suitable as detectable tags for the present invention and the corresponding substrates include, without limitation:
- Alkaline phosphatase:
   o Chromogenic substrates: substrates based on p-nitrophenyl phosphate (p-NPP), 5-bromo-4-chloro-3-indolyl phosphate/nitroblue tetrazolium (BCIP/NBT), Fast-Red/naphthol phosphate-AS-TS
   o Fluorogenic substrates: 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), diazonium salts of Fast Blue BB, Fast Red TR, or Fast Red Violet LB.
- Peroxidases:
   o Chromogenic substrates based on 2,2-azinobis(3-ethylbenzothiazoline-6-sulfonic) acid (ABTS), o-phenylenediamine (OPT), 3,3',5,5'-tetramethylbenzidine (TMB), o-dianisidine, 5-aminosalicylic acid, 3-dimethylaminobenzoic acid (DMAB) and 3-methyl-2-benzothiazolinehydrazone (MBTH), 3-amino-9-ethylcarbazole (AEC) and 3,3'-diaminobenzidine (DAB) tetrahydrochloride.
   o Fluorogenic substrates: 4-hydroxy-3-methoxyphenylacetic acid, reduced phenoxazines, reduced benzothiazines, including Amplex® Red reagent, Amplex UltraRed reagent and reduced dihydroxanthenes.
- Glycosidases:
   ∘ Chromogenic substrates: o-nitrophenyl-β-D-galactoside (O-NPG), p-nitrophenyl-β-D-galactoside and 4-methylumbelliphenyl-β-D-galactoside (MUG) for β-D-galactosidase.
   ∘ Fluorogenic substrates: resorufin β-D-galactopyranoside, fluorescein digalactoside (FDG), fluorescein diglucuronide, 4-methylumbelliphenyl-β-D-galactopyranoside, carboxyumbelliferyl-β-D-galactopyranoside and fluorinated coumarin β-D-galactopyranosides.
- Oxidoreductases (luciferase):
   ∘ Luminescent substrates: luciferin.

In an alternative embodiment, the detectable tag is a fluorescent dye. As used in the present invention, the term "fluorescent dye" or "fluorescent compound" refers to all those compounds which absorb light at a determined wavelength or wavelength range and emit light at a different wavelength or wavelength range. Fluorescent family dyes according to the invention include, without limitation, acridine dyes, cyanine dyes, fluorine dyes, oxazin dyes, phenanthridine dyes and rhodamine dyes. Fluorescent compounds suitable for their use in the present invention include but are not limited to ethidium bromide, SYBR Green, fluorescein isothiocyanate (FITC), tetramethyl rhodamine isothiol (TRIT), 5-carboxyfluorescein, 6-carboxyfluorescein, fluorescein, HEX (6-carboxy-2',4,4',5',7,7'-hexachlorofluorescein), Alexa488, Oregon Green 488, Oregon Green 500, Oregon Green 514, Joe (6-carboxy-4',5'-dichloro-2',7'-dimethoxyfluorescein), 5-carboxy-2',4',5',7'-tetrachlorofluorescein, 5-carboxyrhodamine, rhodamine, tetramethylrhodamine (Tamra), Rox (carboxy-X-rhodamine), R6G (rhodamine 6G), phthalocyanines, azomethines, cyanines (Cy2, Cy3 and Cy5), Texas Red, Princeston Red, BODIPY FL-Br2, BODIPY 530/550, BODIPY TMR, BODIPY 558/568, BODIPY 564/570, BODIPY 576/589, BODIPY 581/591, BODIPY TR, BODIPY 630/650, BODIPY 650/665, DABCYL, Eosin, Erythrosine, ethidium bromide, green fluorescent protein (GFP) and the analogs thereof, inorganic fluorescent labels based on semiconductor nanocrystals (Quantum dot), fluorescent labels based on lanthanides such as Eu3+ and Sm3+ and the like.

In an alternative embodiment, the detectable tag is a luminescent compound. Exemplary luminescent compounds comprise, without limitation, Qdot™ nanoparticles supplied by the Quantum Dot Corporation, Palo Alto, CA.

### 2. Pharmaceutical composition of the invention

In another aspect, the present invention relates to a pharmaceutical composition comprising a therapeutically effective amount of the agent according to the invention, together with at least one pharmaceutically acceptable excipient or carrier.

Said combination of the agent of the invention and at least one pharmaceutically acceptable excipient may be found as a prodrug, salt, solvate or clathrate, whether in an isolated dosage form or in combination with additional active agents. Preferred excipients to be used in the present invention include sugars, starches, celluloses, gums and proteins. In a particular embodiment, the pharmaceutical composition of the invention will be formulated as a solid pharmaceutical dosage form (for example tablets, capsules, coated tablets, granules, suppositories, sterile crystalline or amorphous solids that can be reconstituted to provide liquid forms, etc.), liquid dosage form (for example solutions, suspensions, emulsions, elixirs, lotions, ointments, etc.) or semisolid dosage form (gels, pomades, creams and the like). The pharmaceutical compositions of the invention may be administered by any route, including, without limitation, oral, intravenous, intramuscular, intra-arterial, intramedullary, intrathecal, intraventricular, transdermal, subcutaneous, intraperitoneal, intranasal, enteric, topical, sublingual, or rectal route. A review of the different dosage forms of active ingredients and excipients to be used and their manufacturing processes is provided in "Tratado de Farmacia Galénica", C. Faulí and Trillo, Luzán 5, S.A. de Ediciones, 1993 and in Remington's Pharmaceutical Sciences (A.R. Gennaro, Ed.), 20th edition, Williams & Wilkins PA, USA (2000). Examples of pharmaceutically acceptable vehicles are known in prior art and include phosphate buffered saline solutions, water, emulsions, such as oil/water emulsions, different types of humectants, sterile solutions, etc. The pharmaceutical compositions that comprise said vehicles may be formulated by conventional processes which are known in prior art.

The pharmaceutical composition of the invention may be administered by different routes, for example, by intravenous, intraperitoneal, subcutaneous, intramuscular, topical, intradermal, intranasal or intrabronchial route, and can be administered locally, or systemically or directly at the target site.

Topical administrations are preferred for a preventive effect of the pharmaceutical composition. Intravenous or oral administrations are preferred for a curative effect. The present pharmaceutical compositions may further comprise additional microbicidal agents directed against a wide variety of bacteria, viruses, fungi, parasites, and spermicide. The present pharmaceutical composition may be combined with other active ingredients, such as antivirals, antibiotics, immunomodulators or vaccines.

The person skilled in the art will appreciate that the nature of the excipient in the pharmaceutical composition of the invention will depend to a great extent on the administration route. In the case of the pharmaceutical compositions formulated for their oral (or topical) use, a pharmaceutical composition according to the invention normally contains the pharmaceutical composition of the invention mixed with one or more pharmaceutically acceptable excipients. These excipients can be, for example, inert fillers or diluents, such as sucrose, sorbitol, sugar, mannitol, microcrystalline cellulose, starches, including potato starch, calcium carbonate, sodium chloride, lactose, calcium phosphate, calcium sulfate or sodium phosphate; crumbling agents and disintegrants, for example cellulose derivatives, including microcrystalline cellulose, starches, including potato starch, sodium croscarmellose, alginates or alginic acid and chitosans; binding agents, for example sucrose, glucose. sorbitol, acacia, alginic acid, sodium alginate, gelatin, starch, pregelatinized starch, microcrystalline cellulose, aluminum magnesium silicate, sodium carboxymethylcellulose, methylcellulose, hydroxypropyl methylcellulose, ethylcellulose, polyvinylpyrrolidone, polyvinyl acetate or polyethylene glycol, and chitosans; lubricating agents, including glidants and antiadhesive agents, for example magnesium stearate, zinc stearate, stearic acid, silicas, hydrogenated vegetable oils or talc.

In the case of pharmaceutical compositions for their percutaneous or topical administration, the composition is formulated, for example, in the form of emulsions, ointments, solutions or dyes, powders, or in other forms, for example in the form of aerosols or foams. Liquid, semisolid and solid pharmaceutical preparations are suitable for the use according to the invention of the compounds mentioned, in particular solutions, creams, ointments, powders and gel preparations, in which the latter is preferably used due to its increased release of the active compound.

In a particular preferred embodiment, the pharmaceutical compositions of the invention is formulated for topical administration and applied topically, i.e., directly to the skin and to other epithelial surfaces of the body.

The pharmaceutical composition object of the present invention includes all types of solid, semisolid and fluid compositions. Compositions of particular relevance are, for example, pastes, ointments, hydrophilic ointments, creams, gels, hydrogels, solutions, emulsions, suspensions, lotions, liniments, shampoos, jellies, soaps, bars, sprays, powders, films, foams, pads, sponges (for example, collagen sponges), pads, dressings (such as, for example, wound absorbent dressings), potions, bandages, plasters and transdermal release systems.

In a preferred embodiment of the invention the pharmaceutical composition is a cream.

The pharmaceutically acceptable excipients can include solvents, buffering agents, preservatives, wetting agents, chelating agents, antioxidants, stabilizers, emulsifiers, suspending agents, gel-forming agents, bases for ointments, penetration enhancers, perfumes and skin protective agents.

Examples of solvents are, for example, water, alcohols, vegetable or marine oils (for example, edible oils, such as almond oil, castor oil, cocoa butter, coconut oil, maize oil, cotton oil, linseed oil, olive oil, palm oil, peanut oil, poppy oil, rapeseed oil, sesame oil, soy oil, sunflower oil and tea oil), mineral oils, fatty oils, liquid paraffin, polyethylene glycols, propylene glycols, glycerol, liquid polyalkylsiloxanes and mixtures thereof.

Examples of buffering agents are, for example, citric acid, acetic acid, tartaric acid, lactic acid, phosphoric hydrogen acid, diethylamine, etc.

Suitable examples of preservatives for use in the pharmaceutical compositions are parabens, such as methyl, ethyl or propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalkonium chloride and benzyl alcohol, or mixtures of preservatives.

Examples of wetting agents are glycerine, propylene glycol, sorbitol, lactic acid, urea and mixtures thereof. Examples of chelating agents are sodium EDTA and citric acid.

Examples of antioxidants are butylated hydroxyanisole (BHA), ascorbic acid and derivatives thereof, tocopherol and derivatives thereof, cysteine and mixtures thereof.

Examples of emulsifiers are natural gums, for example acacia gum or gum tragacanth; natural phosphatides, for example soy lecithin; sorbitan monooleate derivatives; wool fats; wool alcohols; sorbitan esters; monoglycerides; fatty alcohols; fatty acid esters (for example, fatty acid triglycerides), and mixtures thereof.

Examples of suspending agents are, for example, cellulose and cellulose derivative, such as, for example, carboxymethylcellulose, hydroxyethylcellulose, hydroxypropylcellulose, hydroxypropyl methylcellulose, carrageenan, acacia gum, gum arabic, tragacanth and mixtures thereof.

Examples of gel bases are viscosity-enhancing agents or components capable of collecting exudates from a wound: liquid paraffin, polyethylene, fatty oils, silica or colloidal aluminum, zinc soaps, glycerol, propylene glycol, tragacanth, carboxyvinyl polymers, magnesium and aluminum silicates, Carbopol®, hydrophilic polymers, such as, for example, starch or cellulose derivatives, such as, for example, carboxymethylcellulose, hydroxyethylcellulose and other cellulose derivatives, water-swellable hydrocolloids, carrageenans, hyaluronates (for example, hyaluronate gel possibly containing sodium chloride) and alginates, including propylene glycol alginate.

Examples of ointment bases are, for example, bee wax, paraffin, cetanol, cetyl palmitate, vegetable oils, sorbitan esters and fatty acids (Span), polyethylene glycols and sorbitan ester and fatty acid and ethylene oxide condensation products, for example polyoxyethylene sorbitan monooleate (Tween).

Examples of hydrophobic or water-emulsifying ointment bases are paraffins, vegetable oils, animal fats, synthetic glycerides, waxes, lanoline and liquid polyalkylsiloxanes.

Examples of hydrophilic ointment bases are solid macrogols (polyethylene glycols).

Other examples of ointment bases are triethanolamine soaps, sulfated fatty alcohol and polysorbates.

Examples of powder components are: alginate, collagen, lactose, powder which is capable of forming a gel when it is applied to a wound (it absorbs liquid/exudate of the wound). Normally, the powders intended to be applied in large open wounds must be sterile and the particles present must be micronized.

Examples of other excipients are polymers such as carmellose, carmellose sodium, hydroxypropylmethylcellulose, hydroxyethylcellulose, hydroxypropylcellulose, pectin, xanthan gum, locust bean gum, acacia gum, gelatin, carbomer, emulsifiers such as vitamin E, glyceryl stearates, cetanyl glucoside, collagen, carrageenan, hyaluronates and alginates and chitosans.

In a dental paste or buccal washing formulation or another formulation for applying to the teeth at the dental roots, the composition of the invention can be present in a dissolved state in a carrier with slightly acid pH or as a dispersion in a carrier with neutral pH. It is foreseen that the use of the composition of the invention can form a protective layer on the surface of the teeth.

The aforementioned pharmaceutical compositions for topical administration serve more suitably for direct application to the wounds, or they can be suitable to be applied to, or introduced in, relevant orifice(s) of the body, for example the rectal, urethral, vaginal, aural, nasal or oral orifices. The composition can be simply applied directly to the part to be treated, such as, for example, on the mucosa, or by any appropriate administration route.

The pharmaceutical compositions which have demonstrated to be important in relation to the topical application are those having thixotropic properties, i.e., the viscosity of the composition is affected, for example, if it is shaken or stirred, such that the viscosity of the composition at the time of administration can be reduced and, when the composition has been applied, the viscosity increases such that the composition remains in the application site.

The pharmaceutical compositions suitable for use according to the invention can also be presented in the form of suspensions, emulsions or dispersions. Said pharmaceutical compositions contain the active substance of the enamel in mixture with a dispersing or wetting agent, a suspending agent and/or one or more preservatives and other pharmaceutically acceptable excipients. Said pharmaceutical compositions can also be suitable for use in administering the composition, for example, to an intact or damaged mucosa, such as the oral, buccal, nasal, rectal or vaginal mucosa, or for administration to the intact or damaged skin or to wounds.

Suitable dispersing or wetting agents are, for example, natural phosphatides, for example lecithin or soy lecithin; the condensation products of ethylene oxide with, for example, a fatty acid, a long chain aliphatic alcohol or a partial ester derived from fatty acids and a hexitol or hexitol anhydride, for example polyoxyethylene stearate, polyoxyethylene sorbitol monooleate, polyoxyethylene sorbitan monooleate, etc.

Suitable suspending agents are, for example, natural gums, such as, for example, acacia gum, xanthan gum or tragacanth gum; celluloses, such as, for example, sodium carboxymethylcellulose, microcrystalline cellulose (for example, Avicel® RC 591, methylcellulose); alginates and chitosans, such as, for example, sodium alginate, etc.

Suitable examples of preservatives for use in pharmaceutical compositions according to the invention are the same as those mentioned above.

The pharmaceutical compositions for use according to the invention can also be administered by oral route. The suitable oral compositions can be in the form of a particulate formulation or in the form of a solid, semisolid or fluid dosage form.

The compositions for oral use include solid dosage forms, such as, for example, powders, granules, granulates, sachets, tablets, capsules, effervescence tablets, chewable tablets, cachets, immediate release tablets and modified release tablets, as well as fluid or liquid formulations, such as, for example, solutions, suspensions, emulsions, dispersions and mixtures. Moreover, the composition can be in the form of powders, dispersible powders or granules suitable for preparing an aqueous suspension by adding a liquid medium, such as, for example, an aqueous medium.

Other pharmaceutically acceptable excipients can be colorants, flavoring agents, plasticizers, wetting agents, buffering agents, etc.

In those cases in which the pharmaceutical composition is in the solid unit dosage form (for example, a tablet or a capsule), the unit dosage form can be provided with a coating, such as one or more of the coatings mentioned below.

In those cases in which the composition is in the form of a tablet, capsule or multiple unit composition, the composition or the individual units, or a tablet or a capsule containing the individual units, can be coated, for example, with a sugar coating, a film coating (for example, based on hydroxypropylmethylcellulose, methylcellulose, methylhydroxyethylcellulose, hydroxypropylcellulose, carboxymethylcellulose, acrylate copolymers (Eudragit), polyethylene glycols and/or polyvinylpyrrolidone) or an enteric coating (for example, based on methacrylic acid copolymer (Eudragit), cellulose acetate phthalate, hydroxypropylmethylcellulose phthalate, hydroxypropylmethylcellulose acetate succinate, polyvinyl acetate phthalate, lacquer gum and/or ethylcellulose). Moreover, a time delay material such as, for example, glyceryl monostearate or glyceryl distearate, can be used.

For application to the rectal or vaginal mucosa, the suitable compositions according to the invention include (emulsion or suspension type) suppositories, enemas and rectal gelatin capsules (solutions or suspensions). As pharmaceutically acceptable suitable suppository bases cocoa butter, esterified fatty acids, glycerin gelatin and several water-soluble or -dispersible bases, such as polyethylene glycols and polyoxyethylene sorbitan esters and fatty acids, are included. Several additives, such as, for example, enhancers or surfactants can be incorporated. In a particular embodiment, the composition of the invention is a vaginal cream, as a semi-solid preparation suitable for application to the vaginal tract. Various classes of excipients or vehicles known in the art can be used in its preparation. The excipients comprise materials of naturally occurring or synthetic origin that do not adversely affect the components of the formulation. Suitable carriers for use herein include but are not limited to purified water, white soft paraffin, mucoadhesive polymers, liquid paraffin, polysorbate 60, sorbitan stearate silicone, waxes, petroleum, jelly, polyethylene glycol, and a variety of other materials, depending on the specific type of formulation used.

For application to the nasal mucosa as well as to the oral mucosa, suitable compositions according to the invention are sprays and aerosols for inhalation. In a typical nasal composition, the active substance of the enamel is present in the form of a particulate formulation possibly dispersed in a suitable carrier. The pharmaceutically acceptable carriers and excipients and possibly any other pharmaceutically acceptable material present in the composition, such as diluents, enhancers, flavoring agents, preservatives, etc., are all selected according to conventional pharmaceutical practice in a way that those persons skilled in the art of formulating pharmaceutical products understand.

In a pharmaceutical composition for use according to the invention on skin or mucosa, a composition of the invention is generally present in a concentration of approximately 0.01% to approximately 99.9% w/w. The amount of composition applied will normally result in an amount of total agent of the invention per cm² of area of wound/skin/tissue corresponding to approximately 0.01 mg/cm² to approximately 20 mg/cm², such as approximately 0.1 mg/cm² to approximately 15 mg/cm².

The amount of the composition applied depends on the concentration of the composition of the invention and on the release rate of the active substance of the enamel from the composition, but it is generally in a range corresponding to approximately 15-20 mg/cm² at most. In those cases in which the composition of the invention is administered in the form of a fluid composition, the concentration of the active substance of the enamel in the composition is in a range corresponding to approximately 0.1 to approximately 50 mg/ml. Higher concentrations are in some cases desirable and can also be obtained as a concentration of at least approximately 100 mg/ml.

The concentration of the composition of the invention in the pharmaceutical composition depends on the concentration of the composition, on its strength, on the severity of the disease which is to be prevented or treated and on the age and condition of the patient. The methods applied for the selection of relevant concentrations of the composition of the invention in the pharmaceutical composition are well known for a person skilled in the art and can be carried out according to guidelines for Good Clinical Practice (GCP) or Investigational New Drug (IND) Exemption regulations, according to what is described, for example, in the International Standard ISO/DIS 14155 for clinical investigation of medical devices, 1994, and "ICH" (International Conference on Harmonization): harmonized tripartite guideline for Good Clinical Practice, Brookwood Medical Publications, Ltd., Surrey, UK, 1996. A person skilled in the art will be able to, by means of the use of the methods described in standard textbooks, of the guidelines and of the regulations described above, as well as by means of the use of general common knowledge within this field, select the exact dosage regimen to be performed for the composition of the invention and/or select other active substances and dosage forms using only routine experimenting processes.

The pharmaceutical preparations according to the invention are prepared in a way known by itself and familiar to persons with experience in the art and pharmaceutically acceptable inert inorganic and/or organic carriers are used in addition to the agent according to the invention and/or physiological tolerable salts thereof.

The compositions of the present invention can also be used as spermicides. These compositions then comprise spermicides and may be incorporated into contraceptive devices such as condoms, sponges, vaginal inserts, contraceptive films, diaphragms, suppositories, contraceptive patches or sustained release devices. For use as spermicides, these compositions of the invention can be applied alone; with other microbicides; and with or incorporated into the contraceptive devices described above.

The spermicidal property may be the inherent property of the compound of the invention or an additional spermicidal agent may be added. Microbicide compositions of the present invention are particularly useful for dramatically preventing and/or treating HIV and/or HCV co-infections or diseases, which are associated with HIV and/or HCV, respectively. Preferred subjects are individuals who are exposed to HIV and/or HCV but have not been infected by it yet. For vaginal heterosexual intercourse, the formulations of the invention may be applied into the vagina prior to intercourse. For anal intercourse (heterosexual or homosexual), the formulations of the invention may be inserted into the rectum prior to intercourse. For either vaginal or anal intercourse, the present topical formulations such as the gel formulations described herein may also act as a lubricant. For added protection it is generally preferred that the present the formulations of the invention be applied before intercourse or other sexual activity and that, if appropriate, a condom be used. For even further protection, the present topical formulations such as the gel formulations described herein can be applied as soon as possible after completion of the sexual activity.

The pharmaceutical compositions according to the invention can also be used for mucosal protection of the respiratory airways. For this purpose, the pharmaceutical compositions may be conveniently formulated as nasal sprays, which are particularly useful for preventing and/or treating diseases caused by enveloped virus that are transmitted by droplet contact through coughing/sneezing, such as for example influenza virus (*Orthomyxoviridae* family, causing influenza), parainfluenza virus (*Paramyxoviridae* family, causing croup, pneumonia, bronchiolitis and common cold), and respiratory syncytial virus (*Paramyxoviridae* family, causing influenza-like syndrome, pneumonia and bronchiolitis). The pharmaceutical compositions suitable for this use may be presented in the form of suspensions, emulsions or dispersions, as described above.

### Uses of the agent and composition of the invention

In a further aspect, the invention relates to an agent according to the invention as described previously or to a composition according to the invention as described previously for use in medicine.

Alternatively, the invention relates to the use of an agent according to the invention as described previously or to a composition according to the invention as described previously for the manufacture of a medicament.

In a still further aspect, the invention relates to an agent according to the invention as described previously or to a pharmaceutical composition according to the invention as described previously for use in the prevention and/or treatment of a viral infection in a subject. Alternatively, the invention relates to the use of an agent according to the invention as described previously or to the use of a pharmaceutical composition according to the invention as described previously in the manufacture of a medicament for the prevention and/or treatment of a viral infection in a subject. Alternatively, the invention relates to a method for the prevention and/or treatment of a viral infection in a subject in need thereof comprising administering to said subject a therapeutically effective amount of the agent of the invention as described previously or of the pharmaceutical composition of the invention as described previously.

The agent of the invention has been described previously, as well as the pharmaceutical composition of the invention comprising said agent.

In a particular embodiment, the viral infection is caused by an enveloped virus. In a more particular embodiment, the viral infection is caused by a virus selected from the group comprising a virus of the *Retroviridae* family, a virus from the *Herpesviridae* family, and a virus of the *Flaviviridae* family. In a more particular embodiment, the retrovirus is selected from the group comprising human immunodeficiency virus 1 (HIV-1), human immunodeficiency virus 2 (HIV-2), human T-lymphotropic virus I (HTLV-I) and human T-lymphotropic virus II (HTLV-II). In a more particular embodiment, the retrovirus is selected from the group comprising HIV-1 and HIV-2, more preferably HIV-1. In a particular alternative embodiment, the herpesvirus is selected from the group comprising cytomegalovirus (CMV), herpes simplex virus types 1 and 2 (HSV-1 and HSV-2), and Epstein-Barr virus (EBV). In a particular alternative embodiment, the virus is selected from a virus of the *Flaviviridae* family. In a more particular embodiment, the *Flaviviridae* virus is selected from the group comprising Human Hepatitis C Virus (HCV), Yellow fever virus, West Nile virus and Dengue Fever virus.

In a particular embodiment, the viral infection is caused by a virus selected from the group comprising human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus (HSV), Epstein-Barr virus (EBV), respiratory syncytial virus (RSV), human cytomegalovirus, human T-lymphotropic virus I (HLTV I) and human T-lymphotropic virus II (HLTV II). In a preferred embodiment, the viral infection is caused by HIV, more preferably by HIV-1. In another preferred embodiment, the viral infection is caused by HCV.

In a further aspect, the invention relates to an agent according to the invention as described previously and to a pharmaceutical composition according to the invention as described previously for use in the prevention of transmission of HIV and/or HCV infection, wherein the transmission is via sexual intercourse or related intimate contact between partners and wherein said agent or said composition is applied to the site where sexual intercourse or related intimate contact between partners takes place or is to take place. Alternatively, the invention relates to the use of an agent according to the invention as described previously or to the use of a composition according to the invention as described previously in the manufacture of a medicament for the prevention of transmission of HIV and/or HCV infection, wherein the transmission is via sexual intercourse or related intimate contact between partners and wherein said agent or said composition is applied to the site where sexual intercourse or related intimate contact between partners takes place or is to take place. Alternatively, the invention relates to a method for the prevention of transmission of HIV and/or HCV infection in a subject in need thereof, wherein the transmission is via sexual intercourse or related intimate contact between partners and comprising administering to said subject on the site where sexual intercourse or related intimate contact between partners takes place or is to take place a therapeutically effective amount of an agent according to the invention as described previously or a composition according to the invention as described previously.

While the agent of the invention as described previously and the composition of the invention as described previously for their use in the prevention and/or treatment of viral infection are particularly useful for inhibiting viral infections, particularly HIV and/or HCV and infections by other enveloped virus, in human beings, they can also be useful for veterinary purposes for infectious diseases in animals, such as: dogs, cats, birds, horses, cows, sheep, swine, and other farm animals, as well as rodents and other animals.

The invention relates as well to the following aspects:
1. An agent comprising one unit, two units or four units, wherein each of said units comprises:
   (i) an oligonucleotide comprising at least four consecutive guanines (G), wherein said at least four consecutive G form an intra-unit (intra-molecular) or an inter-unit (inter-molecular) G-quadruplex, wherein said G-quadruplex binds to a viral envelope glycoprotein,
   and wherein at least one of said units comprises:
   (ii) a lipophilic substituent moiety, wherein said lipophilic molecule anchors to a viral envelope or to the membrane of a cell susceptible of being infected by a virus, and
   (iii) a linker, wherein said linker links the 5' or 3' end of the oligonucleotide in (i) to the lipophilic substituent moiety in (ii),
   wherein said agent has antiviral activity.
2. An agent according to aspect 1, wherein the oligonucleotide in (i) comprises the nucleotide sequence

   *5'- X₁ X₂ G G G (G)ᵢ X₃* (SEQ ID NO:8) *(tail) - 3'*

   wherein
   - X₁ may be present or absent, and represents any nucleotide,
   - X₂ represents T, A or C, preferably T or A,
   - i is an integer between 1 and 4,
   - X₃ represents T, A or C, preferably T or A,
   - (tail) represents a nucleotide sequence, comprising between 0 and 23 nucleotides.
3. An agent according to any of aspects 1 or 2, wherein said agent comprises one unit, and wherein said unit is arranged in a monomolecular G-quadruplex.
4. An agent according to aspect 3, wherein the oligonucleotide tail comprises three repetitions of at least three consecutive G, wherein each of said at least three consecutive G is separated from the following at least three consecutive G by at least one nucleotide.
5. An agent according to aspect 4, wherein the oligonucleotide comprises the sequence 5'-TTGGGGATAGGGATAGGGATAGGGATA-3'. (SEQ ID NO: 1).
6. An agent according to any of aspects 1 or 2, wherein said agent comprises two units, and wherein said two units are arranged in a bimolecular G-quadruplex.
7. An agent according to aspect 6, wherein the oligonucleotide tail comprises at least three consecutive G.
8. An agent according to any of aspects 1 or 2, wherein said agent comprises four units, and wherein said four units are arranged in a tetramolecular parallel G-quadruplex.
9. An agent according to aspect 8, wherein the oligonucleotide tail does not comprise at least two consecutive G.
10. An agent according to any of aspects 8 or 9, wherein the oligonucleotide comprises the sequence 5'-TTGGGGGGTAC-3' (SEQ ID NO: 2).
11. An agent according to aspect 10, wherein the oligonucleotide comprises the sequence 5'-TTGGGGGGTACAGTGCA-3' (SEQ ID NO: 3).
12. An agent according to any of aspects 1, 2 or 6 to 11, wherein the lipophilic substituent moiety comprised by one of the units of the agent is the same as the lipophilic substituent moiety comprised by the other unit(s) of the agent.
13. An agent according to any of aspects 1, 2 or 6 to 11, wherein the lipophilic substituent moiety comprised by one of the units of the agent is different from the lipophilic substituent moiety comprised by the other unit(s) of the agent.
14. An agent according to any of aspects 1 to 13, wherein the lipophilic substituent moiety is a lipid selected from the group consisting of a fatty acid, an acylglyceride, a phospholipid, a glucolipid, a terpenoid and a steroid, or comprises a C8-C30 alkyl substituent.
15. An agent according to aspect 14, wherein the lipid is selected from the group consisting of stearic acid and cholesterol.
16. An agent according to any of aspects 1 to 15, wherein the linker is selected from the group consisting of glycerol, serinol, threoninol, and hydroxyprolinol.
17. An agent according to any of aspects 1 to 16, wherein the oligonucleotide comprises modifications in the nucleobases, sugars and/or internucleotide linkages.
18. An agent according to aspect 17 wherein the internucleotide linkages are selected from the group consisting of phosphodiester links and phosphorothioate links.
19. An agent according to any of aspects 1 to 18, wherein the oligonucleotide is DNA, RNA, LNA, PNA or a mixture thereof.
20. An agent according to any of aspects 1 to 19, wherein the oligonucleotide additionally comprises a capping group, wherein said capping group is linked to the end of the oligonucleotide which is not attached to the linker, and wherein said capping group is selected from the group consisting of poly-L-lysine, propylamine, biotine and a fluorescent dye.
21. An agent according to any of aspects 1 to 20, wherein the envelope glycoprotein is an envelope glycoprotein from an enveloped virus.
22. An agent according to aspect 21, wherein the enveloped virus is selected from the group consisting of human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus (HSV), Epstein-Barr virus (EBV), respiratory syncytial virus (RSV), human cytomegalovirus, human T-lymphotropic virus I (HLTV I), human T-lymphotropic virus II (HLTV II), West Nile virus, Dengue virus, and Chikungunya virus.
23. An agent according to aspect 22, wherein the virus is selected from the group consisting of HIV and HCV.
24. An agent according to aspect 23, wherein the HIV envelope glycoprotein is gp120.
25. An agent according to aspect 23, wherein the HCV envelope glycoprotein is selected from the group consisting of E1 and E2.
26. A pharmaceutical composition comprising a therapeutically effective amount of an agent according to any of aspects 1 to 25, together with at least one pharmaceutically acceptable excipient or carrier.
27. A pharmaceutical composition according to aspect 26, wherein the composition is formulated for topical administration.
28. An agent according to any of aspects 1 to 25 or a pharmaceutical composition according to any of aspects 26 or 27 for use in medicine.
29. An agent according to any of aspects 1 to 25 or a pharmaceutical composition according to any of aspects 26 or 27 for use in the prevention and/or treatment of viral infection in a subject, wherein said viral infection is caused by an enveloped virus.
30. An agent or a pharmaceutical composition for use according to aspect 29, wherein the enveloped virus is selected from the group consisting of human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus (HSV), Epstein-Barr virus (EBV), respiratory syncytial virus (RSV), human cytomegalovirus, human T-lymphotropic virus I (HLTV I), human T-lymphotropic virus II (HLTV II), West Nile virus, Dengue virus and Chikungunya virus.
31. An agent or a pharmaceutical composition for use according to aspect 30, wherein the enveloped virus is selected from the group consisting of HIV and HCV.

The invention is described below by the following examples, which must be considered as merely illustrative and in no case limiting of the scope of the present invention.

### EXAMPLES

### Materials and Methods

### 1. Synthesis of the oligonucleotides and conjugation with a lipid group

### 1.1. Synthesis of ODN-Lipid (C18) conjugate with 3' end-modification

The synthesis of a glyceryl-based lipid modification is outlined in Figure 1. Mesylate **(7),** which was easily obtained from the respective alcohol **(6),** and was reacted with solketal **(1).** The nucleophilic substitution gave **(2)** in good yields after purification by flash chromatography. Deprotection of the acetonide group was carried out using p-methylbenzenesulfonic or tosyl acid pTsOH in methanol thereby obtaining the corresponding diol **(3)** with good yield. Subsequently, primary alcohol was selectively protected with monomethoxytrityl chloride (MMTr-Cl) in pyridine. After purifying by flash chromatography, MMTr-protected lipid **(4)** was isolated with a moderate yield. Finally, we introduced a succinic anhydride unit to the secondary alcohol prior to obtaining Controlled-Pore-Glass (CPG) solid-support **(5)** modified with the corresponding lipid C18 moiety.

Once CPG solid-support **(5)** was in our hands, automatic DNA synthesis was carried out according to established protocols. The sequence GQVIR17 d(5'-TTGGGGGGTACAGTGCA-3') (SEQ ID NO: 3) was introduced by modifying the 3'-termini with our lipid **(3).** The modified oligonucleotide **(8)** was purified using DMTon based-protocols. Then the last DMT group was removed in acid conditions (Fig. 2). Finally, the modified oligonucleotide was desalted (Sephadex, NAP-10) and characterized by HPLC and MALDI-TOF mass spectrometry. A similar approach was used for the preparation of 5'Cy5-GQVIR17-3'C18 **(9).** In this case in addition to build the sequence (SEQ ID NO: 3) on the solid support modified with the corresponding lipid C18 moiety, the Cy5 dye was added at the 5'-end of the sequence using the corresponding Cy5 phosphoramidite derivative (see below).

### 1.2. 5' addition of the Cy5 moiety

In order to introduce a Cy5 dye at 5'-termini of the respective G4-ON, we used a labile protecting group like G(dmf) phosphoramidite. The introduction of Cy5 phosphoramidite at 5'-termini into the lipid oligonucleotide (8) was manually carried out. The deprotection of the protecting groups in ammonia (32%) at room temperature was carried out for 6 h which provided the corresponding fluorescence modified oligonucleotide-lipid conjugate (9) (Fig. 2) which was characterized by MALDI-TOF mass spectrometry.

### 1.3. Synthesis of ODN modified with cholesterol at 3'-termini

Commercially available Cholesterol CPG solid-support (Link Technologies) was used in order to introduce a cholesterol moiety at 3'-termini using the sequence GQVIR17 d(5'-TTGGGGGGTACAGTGCA-3') (SEQ ID NO: 3). The modified oligonucleotide was purified according to the same protocols previously mentioned.

Furthermore, we also synthesized the unmodified oligonucleotide GQVIR17 as well as an ODN of sequence d(5'-TTGAAAGGTACAGTGCA-3') GQVIR17(A4-6) (SEQ ID NO: 4) in order to obtain the corresponding oligonucleotide controls (see Table 1 in the Example 1). All compounds were characterized by HPLC and confirmed by MALDI-TOF mass spectrometry.

### 2. Formation of G-Quadruplexes

The dry ODNs were dissolved at a concentration of 1.2 mM in a buffer containing 20 mM Tris Acetate pH 7.0 and 50mM Potassium Acetate. The amount of oligonucleotides was quantified with a Nanodrop spectrophotometer (Thermo Scientific). After a vigorous shaking for one minute, the ODN were centrifuged on a microcentrifuge for 5 seconds at 6000 rpm and stored at 25°C for at least 20 days. G4-ON formation was followed by 10-12% non-denaturing PAGE according to (Lyonnais et al, 2003, cited *supra*) and Circular Dichroism (CD). CD spectra were recorded on a Jasco J-810 spectropolarimeter equipped with a Julabo F-25/HD temperature control unit. Hellma quartz cells (10 mm path length, 3mL volume) were used. Compounds were used without further purification when the ODN monomers became undetectable vs. maximum intensity of the G4-ON band by gel electrophoresis. G4-ON concentration was estimated as a quarter of the total ODN concentration. Once formed, the G4-ONs and the control ODN were stored at -20°C at a concentration of 800µM (ODN monomers thus 200µM G4-ONs) in 20mM Tris-Acetate pH7.0, 50mM Potassium Acetate.

### 3. Evaluation of anti-HIV activity

### Plasmids, viruses and cells

The vector pNL4.3-Ren was generated by cloning the renilla luciferase gene in the nef site of HIV-1 proviral clone pNL4.3. Vesicular Stomatitis Virus (VSV)-pseudotyped HIV-1 was produced by co-transfection of plasmids pNL4.3-Δenv-Luc, which does not express the HIV envelope, with pcDNA3-VSV, encoding the G protein of the vesicular stomatitis virus (VSV). pcDNA3-VSV was obtained from F. Arenzana, Pasteur Institute, Paris. HIV-1LAI isolate was obtained from the Centre for AIDS Reagents, NIBSC, UK.

SupT1 cells (NIH AIDS Research and Reference Reagent Program) were cultured in RPMI 1640 medium containing 10% (v/v) fetal bovine serum (FBS), 2 mM L-glutamine, penicillin (50 IU mL-1) and streptomycin (50 µg mL-1) (all Whittaker M.A. Bio-Products). PM1 cells (NIH AIDS Research and Reference Reagent Program, Cat# 3038) were maintained with RPMI medium supplemented with 10% heat-inactivated FCS and 1% of penicillin-streptomycin. 293T cells were cultured in DMEM medium containing 10% (v/v) FBS, 2mM L-glutamine, penicillin (50 IU/mL) and streptomycin (50 µg mL-1). TZM-bl cells were obtained from the NIH AIDS Research and Reference Reagent Program (Cat# 8129) and maintained in DMEM supplemented with 10% heat-inactivated FCS, HEPES 25 mM and 0.5% gentamycin. Cells were cultured at 37 °C in a 5% CO₂ humidified atmosphere and splinted twice a week.

### Production of recombinant viruses and infectivity assays.

**Assay A.** Recombinant virus stocks were obtained from calcium phosphate transfection of plasmid pNL4.3-Ren on 293T cells. Virus stocks were then stored at - 80°C until use. The infectivity assay of HIV was performed in triplicate by infecting SupT1 cells using 96-well culture plate in the presence or absence of drugs, using 100µL of virus stock to infect 1x10⁵ cells per well in a final volume of 200µL. HIV replication was evaluated 72 hours post-infection using the "Renilla luciferase assay system" (Promega). Briefly, cells were lysed and relative luminescence units (RLUs) were obtained in a luminometer (Berthold Detection Systems) after the addition of substrate to cells extracts. After 72 h, cell viability was assessed by a CellTiter-Glo® Luminescent Cell Viability Assay (Promega, Madison, USA). Viruses were also quantified by determining the concentration of p24 in the supernatant with an antigen (Ag) capture assay (ELISA; Innogenetics NV, Gent, Belgium).

**Assay B.** HIV-1 Lai virus was propagated in PM1 cells and titrated in TZM-bl cells as described previously (Montefiori, 2005, Curr Protoc Immunol, Chapter 12, Unit 12.11). One mL aliquots of virus stocks were stored at -80°C until use. To monitor compound-related toxicity in parallel, TZM-bl cells were left uninfected and incubated with test compounds. For drug-response assays TZM-bl cells were plated (10⁴ cells/ well) in Nunc® MicroWell 96 well optical bottom plates (Sigma) and incubated for 1 h with increasing concentrations of test compounds in 10-fold dilutions or with the buffer as negative control in triplicates. After drug incubation, cells were infected with HIV-Lai and 48 h after infection luciferase activity was measured using Britelite PlusTM (PerkinElmer, Waltham, USA). In parallel, cell viability of TZM-bl cells was determined with an ATP quantification method using the commercial kit CellTiter-Glo® Luminescent Cell Viability Assay (Promega, Madison, USA). Mean luciferase values were normalized to untreated controls.

For the cell viability in HeLa cells, 9,500 cells/well were seeded in 96-well plates and cultured overnight in DMEM with 7% FCS. Then, cells were washed with PBS and treated with increasing concentration of GQHIV17-3'C18 in 50 µL optiMEM (Gibco) for 4 hours. Thereafter, 150 µL of medium with 7% FCS was added and 24 and 48 hours after viability of cells was measured using the CellTiter 96 Aqueous Nonradioactive Cell Proliferation Assay (MTS-assay, Promega) according to the manufacturer's instructions.

### Cell Microscopy with HeLa cells

Forty thousand HeLa cells were seeded in an 8-well Ibidi microslide (Biovalley) in 300 µL of DMEM (Gibco) with 7% FCS and cultured overnight. Then, the medium was discarded and cells were washed one time with HBSS (Gibco). Cells were treated with 1 µM of 5'Cy5-GQVIR17-3'C18 in optiMEM (Gibco) for 4 h at 37°C. Then, the medium was replaced with DMEM without Phenol Red (Gibco) and cells were observed under a Zeiss Axio Observer.Z1 microscope equipped with a 37°C incubation room. Images were taken with a OrcaR2 (Hamamatsu) camera, using a 63x oil immersion objective with a numerical aperture of 1,4 and a (50 Cy5 shift free 488050 9901) far red filter (EX BP 640/30, EM BP 690/50). Acquisitions were made with the AxioVision4.8 software and presented images are the projections of all z stacked signals (0,230 µm between each images), analyzed with the ImageJ software.

### 4. Evaluation of anti-HCV activity

### Cell Culture and Cell Lines

Huh-7/Scr and Huh7.5.1 Cl.2 cells (Zhong et al., 2006, J Virol 80:11082-93) (kindly provided by F. Chisari, Department of Immunology & Microbial Science, Laboratory of Experimental Virology, La Jolla, CA, USA), and 293T (HEK293T cells, American Type Culture Collection, Manassas, VA, CRL-1573) cells were grown in Dulbecco's modified Eagle medium (DMEM; Invitrogen, Carlsbad, CA) supplemented with 2 mM L-glutamine, 1% non-essential amino acids, 100 U of penicillin per ml, 100 mg of streptomycin per ml, and 10% fetal calf serum (FCS), designated DMEM complete, in an incubator with 5% CO₂ at 37°C.

### Plasmids

The plasmids pFK-Jc1 (Pietschmann et al., 2006, Proc Natl Acad Sci USA 103:7408-13) and pFK-Luc-Jc1 (Koutsoudakis et al., 2006, J Virol 80:5309-20) have been previously described. The subgenomic replicon plasmid pSGR-JFH1 carries a bicistronic construct where a Firefly luciferase gene is expressed via the HCV IRES and an EMCV IRES drives expression of JFH1 non-structural proteins (NS3 to NS5B) (Kato et al, 2003).

The plasmid-encoding strain HC-J6CH (GenBank accession No. AF177036, version 1, 5^{th} September, 2007) E1E2 glycoproteins, named pcDNA3.1-ΔcE1E2-J6CH, has been described elsewhere (Koutsoudakis et al., 2012, PLoS One 7:e52651). The plasmid pTN7-Stopp (kindly provided by M. Dittmar, Blizard Institute, Centre for Immunology and Infectious Disease, London, UK) carries the HIV-1 genome with the following modifications: the Renilla luciferase reporter gene has replaced the nef gene and lacks a functional env gene (Neumann et al., 2005, Virology 333:251-62).

### In vitro transcription

Plasmids carrying Luc-Jc1 constructs were linearized with the MluI enzyme while the plasmid pSGR-JFH1 and the plasmids carrying genotype 1-7/JFH1 chimeric viruses were linearized with the XbaI enzyme. Linearized plasmid DNA was purified with the QIAquick PCR purification kit (Qiagen, Düsseldorf, Germany). Purified DNA was subjected to an in vitro transcription reaction with the MEGAscript® T7 kit (Applied Biosystems, Foster city, CA) according to manufacturer's protocol. RNA from in vitro transcription reaction was purified with the Nucleospin® RNA II kit (Macherey-Nagel, Düren, Germany). The RNA integrity checked by denaturing agarose gel electrophoresis and the concentration was determined by measurement of the optical density at 260 nm. Finally the RNA was kept at -80 °C.

### Electroporation of HCV RNAs, generation of HCVcc stocks and determination of viral titers

Single-cell suspensions of Huh7.5.1 Cl.2 cells were prepared by trypsinization of monolayers and subsequent resuspension with DMEM complete. Cells were washed with phosphate-buffered saline (PBS), counted, and resuspended at 1.5 x 107 cells per ml in Cytomix (van den Hoff et al, 1995) containing fresh 2 mM ATP and 5 mM glutathione. 10 µg of in vitro transcribed RNA was mixed with 400 µl of the cell suspension by pipetting, electroporated and immediately transferred to 10 ml of complete DMEM. Subsequently, the cells were seeded at a density of 3.125 x 10⁵ cells/cm², which corresponds to 2 ml of the cell suspension per well of a six-well plate. Electroporation conditions were 960 µF and 270 V with a Gene Pulser Xcell™ system (Bio-Rad, Munich, Germany) and a cuvette with a gap width of 0.4 cm (Bio-Rad).

For the generation of HCVcc stocks, 10 mL electroporated cells were seeded onto 10-cm dishes. Supernatant from electroporated cells was harvested 96 h post electroporation, cleared by passing through 0.45-µm-pore-size filters and kept at 4 oC up to 3 weeks without significant loss of infectivity as determined by TCID₅₀ estimation of the virus titer.

For the determination of viral titers Huh7/Scr cells were seeded at a concentration of 1.2×10⁴ cells per well in a 96-well plate in a total volume of 200 µl. Twenty-four hours later, serial dilutions of virus containing supernatant were added (6 wells per dilution.) Three days later, cells were washed with PBS and fixed for 20 min with ice-cold methanol at -20°C. After three washes with PBS, NS5A was detected with a 1:2000 dilution of the α-NS5A antibody 9E10 (kindly provided by C. Rice, The Rockefeller University, NY) in PBS supplemented with 5% BSA for 1 h at room temperature. Cells were washed again three times with PBS and bound primary antibodies were detected by incubation in PBS-5% BSA with goat α-mouse IgG-peroxidise conjugated antibody (Sigma-Aldrich, St. Louis, MO) at 1:400 dilution. After 1 h incubation at room temperature, cells were washed three times with PBS; the Vector NovaRED substrate kit (Linaris Biologische Produkte GmbH, Wertheim, Germany) was used for detection of peroxidase. Virus titers (50% tissue culture infective dose per ml (TCID₅₀/ml)) were calculated based on the Spearman and Kärber method (Hamilton, Russo and Thurston, Environ. Sci. Technol., 1977, 11 (7), pp 714-719)

### HCVpp production

HIV-based pseudoparticles bearing HCV glycoproteins were generated by calcium phosphate co-transfection of 293T cells. Briefly, 293T cells were seeded in 10 cm tissue culture dishes (3.6 x 106 cells/dish) and co-transfected with 10 µg of HIV packaging plasmid (pTN7-Stopp] and 10 µg of pcDNA3.1-ΔcE1E2-J6CH by calcium phosphate transfection. The medium (10 ml/plate) was replaced 10 h after transfection. Supernatants containing the pseudo-particles were harvested 24 h later, filtered through 0.45-µm pore-sized membranes, and used in infection assays.

### HCVcc and HCVpp infections

Huh7/Scr cells were seeded on 96 well plates at 1.2 x 10⁴ cells/well, 16 h prior to infections. On the day of infections, cells were treated with increasing concentrations of compounds, then compounds-containing media were removed and cells were infected with Luc-Jc1 virus-compounds mix at a multiplicity of infection (MOI) of ∼0.01-0.03TCID50/cell. Virus-compounds mix were replaced 4 h post infection with fresh media-compounds mix and 72 h after infection cells were assayed for Firefly luciferase activity and the mean relative light units (RLU) were plotted as percentage relative to DMSO for both infectivity and cell viability. Similar protocol was followed for the HCVpp infection but viruses-compounds mix was added to cells for 6 h while 72h post infection cells were assayed for Renilla luciferase activity as described below.

### Luciferase and cytotoxicity (viability) assays

Firefly and Renilla luciferase activities were assayed 72h post infection with the Dual-Glo® Luciferase Assay System while cytotoxicity (viability) assays were carried out with the CytoTox-Glo™ Cytotoxicity Assay (both purchased from Promega Corporation, Madison, WI), according to manufacturer's instructions, using a plate luminometer FLUOstar OPTIMA (BMG LABTECH, Ortenberg, Germany). Mean relative light units (RLU) were plotted as percentage relative to control infections (solvent without compounds) for both infectivity and cell viability. Infections were carried out in duplicates and measured in duplicates (mean ± SEM; n=4). Effective Concentration 50 (EC₅₀) and Cytotoxic Concentration 50 (CC₅₀) were estimated by nonlinear regression of log inhibitor vs. normalized response and used to calculate the Selectivity Index (SI) value

### HCV RNA quantification by RT-qPCR

Viral RNA was isolated from virus-inoculated cells using the Total RNA kit I (OMEGA bio-tek, Norgross, GA) as recommended by the manufacturer. RNA concentration was determined by measurement of the optical density at 260 nm. 20 ng of the total RNA sample was used for quantitative RT-qPCR analysis using a 7500 Real-Time PCR sequence detector system (Applied Biosystems, Waltham, MA). HCV-specific RT-qPCRs were conducted in duplicates for each sample with the qScript™ XLT One-Step RT-qPCR ToughMix®, ROX™ (Quanta Biosciences, Gaithersburg, MD) using the following 5' NTR-specific probe S-292, 5'-6-carboxyfluorescein-CCTGATAGGGTGCTTGCGAGTGCC (SEQ ID NO: 5)-tetrachloro-6-carboxyfluorescein-3'; and primers; S-271, 5'-GCGAAAGGCCTTGTGGTACT-3' (SEQ ID NO: 6); and A-337, 5'-CACGGTCTACGAGACCTCCC -3' (SEQ ID NO: 7) (Biomers, Ulm, Germany). Reactions were performed in three stages by using the following conditions: stage 1, 15 min at 55°C (reverse transcription); stage 2, 1 min at 95°C (heat inactivation of reverse transcriptase and activation of Taq polymerase); and stage 3, 40 cycles of 10 s at 95°C and 1 min 60°C (amplification). The amount of HCV RNA was calculated by comparison to serially diluted in vitro transcripts.

### Statistical analyses

The results are presented as means ± standard errors of the mean (SEM). The statistical comparison between two groups was made by an unpaired-t test. *p value < 0.05, **p value < 0.01 and ***p value < 0.001 were considered to indicate a significant difference.

### Example 1: Oligonucleotides synthesized for this study and their properties

Table 1 summarizes the oligonucleotides synthesized for this study and their ability to form G-quadruplex. Briefly, Lipophilic and unlabeled ODNs were synthesized according to the procedure described in the experimental section and incubated in presence of potassium ions, which favour their formation (Lyonnais et al., 2002, cited *supra*). The formation of parallel G4-ON (which is a slow process, e.g. in weeks) was followed by non denaturing poly-acrylamide gel electrophoresis (PAGE) and circular dichroism (CD) (Fig. 3). As published previously (Lyonnais et al, 2002, cited *supra*), the 17-mer d(5'-TTGGGGGGTACAGTGCA-3') (SEQ ID NO: 3) (GQVIR17) forms single species of parallel tetramolecular G4-ON, as seen with the apparition of a single band of low mobility by gel electrophoresis, as compared with the unfolded monomer (Fig.3a, lane 2). The CD spectra ascertain the formation of G4-DNA in a buffer containing 50mM KCl, with strong positive ellipticity maximum around 265 nm and negative ellipticity minimum at 245 nm, the signature of parallel G-quadruplexes. The addition in 3' of an alkyl chain (C18) (Fig.3a, lane 4) or a cholesterol group (Chol) (Fig.3a, lane 5) did not alter G4-ON formation, as we observed the same up-shifted band than the one observed for GQVIR17 in gel electrophoresis, and the CD spectrums are equivalents (Fig.3b). GQVIR17-3'C18 and GQVIR17-3'Chol were seen to form minor higher order structures separated by PAGE (* in Fig.3a). Substitution of guanines by adenines at position 4-6 in GQVIR17 (SeqVIR17(A4-6)) and its 3'-C18 conjugate (SeqVIR17(A4-6)-3'C18) was used to impair G-quadruplex formation [e.g. by disrupting the GGGGGG series] and to produce the monomeric forms of the ODN and the ODN-lipid conjugates. This was confirmed in Fig. 3a, with shows only a single band for these ODNs (lanes 1 and 3). A shorter 11-mer ODN, GQVIR11-3'C18 d(5'-TTGGGGGGTAC-3'C18) based on d(5'-TTGGGGGGTAC-3') (SEQ ID NO: 2) forming a G-quadruplex was also realized (Fig.3a, lane 6). A fluorescent GQVIR17-3'C18 was obtained by addition of a Cy5 fluorescent dye at the 5' terminus.

**Table 1: Oligonucleotides and ability to form G-quadruplex**

| **Sequence 5'->3'** | **Name** | **Description** |
|---|---|---|
| ODN based on SEQ ID NO: 4 | | |
| TTGAAAGGTACAGTGCA | seqVIR17(A4-6) | ODN unable to form G-quadruplex |
| TTGAAAGGTACAGTGCA-C18 | seqVIR17(A4-6)-3'C18 | ODN (unable to form G-quadruplex) - C18 |

| ODN based on SEQ ID NO: 3 | | |
|---|---|---|
| TTGGGGGGTACAGTGCA | GQVIR17 | G-quadruplex (TTGGGGGGTACAGTGCA)₄ |
| TTGGGGGGTACAGTGCA-C18 | GQVIR17-3'C18 | G-quadruplex (TTGGGGGGTACAGTGCA-C18)₄ |
| TTGGGGGGTACAGTGCA-Chol | GQVIR17-3'Chol | G-quadruplex (TTGGGGGGTACAGTGCA-Chol)₄ |
| Cy5-TTGGGGGGTACAGTGCA-C18 | 5'Cy5-GQVIR17-3'C18 | Fluorescent G-quadruplex (Cy5-TTGGGGGGTACAGTGCA -C18)₄ |

| ODN based on SEQ ID NO: 2 | | |
|---|---|---|
| TTGGGGGGTAC-C18 | GQVIR11-3'C18 | G-quadruplex (TTGGGGGGTAC-C 18)₄ |

| | | |
|---|---|---|
| Abbreviations: C18, linoleic acid; Chol, cholesterol; Cy5, cyanine 5 dye. | | |

### Example 2: Antiviral activity of the compounds against HIV

### 1. Inhibition of HIV infection

The antiviral effect of the compounds on HIV was evaluated in cell cultures using two different recombinant virus assays allowing viral infection to be measured directly (assay A and assay B, details in the experimental section). The assay A used the pNL4.3-Ren HIV vector, expressing the Renilla luciferase reporter gene in the place of the *nef* gene in the pNL4-3 clone. Infectious supernatants were obtained from transfection on 293T cells of plasmids pNL4.3-Ren and these supernatants were used to infect SupT1 cells in the presence or absence of the compounds to be tested. The assay B used TZM-bl cells, containing an integrated Tat-dependent firefly luciferase gene. Upon infection with HIV-1 Lai, the proviral-produced Tat protein mediates the activation of the LTR-driven luciferase gene. Cell viability was followed in parallel in both assays.

Both independent experiments show a reproducible and complete inhibition of HIV viral infection at low µM concentrations of GQVIR17-3'C18 (Figs. 4 and 5). GQVIR11-3'C18 was tested in the assay B and also completely inhibits luciferase expression at a concentration of around 0.8 µM. For GQVIR17-3'C18, we found an EC₅₀ between 50-100 nM in the assays A in four independent experiments. The assay B showed an EC₅₀ of 0.28µM in HIV-1Lai for GQVIR11-3'C18. At a concentration of 1µM, the non-conjugated G4-ON GQVIR17 shows a weaker antiviral activity (EC₅₀ around 1-2µM in both assays), however comparable with previously reported antiviral G4-ONs. The disruption of the G4-ON structure (seqVIR17A(4-6)) completely abrogates the antiviral effect, with or without the 3' addition of the lipophilic anchor C18. The glycerol derivative of linoleic acid C18 alkyl alone has no effect (not shown). Viability assays show an absence of toxicity after 72 hours for all the drugs tested, at concentrations up to 10µM, as reported for other G4-ONs *in vitro.*

Additional experiments using the assay B were performed to compare the antiviral activity of GQVIR17-3'C18 with a commercial compound and between HIV-1 Lai and HIV-2 Rod strain (Fig. 6). We found a similar value (EC₅₀ =0.37µM) for HIV-1 Lai but also an EC₅₀ of 1.18µM for HIV-2. This demonstrates a potent broad-spectrum antiviral effect of GQVIR17-3'C18 against different strains of HIV.

### 2. GQVIR17-3'C18 inhibits HIV entry and interacts with HIV particle

To assess whether the antiviral activity of the compounds is produced before or after HIV transcription, a transfection experiment was performed with GQVIR17-3'C18 and seqVIR17(A4-6)-3'C18. In this experiment the HIV-coding pNL4.3-Ren is transfected into 293T cells in the absence of drugs. After 24h, the medium is replaced and the drug is added. 24h later supernatants are collected, the viruses are quantified and their infectivity is evaluated by infecting SupT1 cells. A control of the activity is performed with Amprenavir (APV), an HIV Protease Inhibitor. As shown in Fig. 7a, inhibition of infection is detected in the transfection experiment only in presence of GQVIR17-3'C18 and APV, while seqVIR17(A4-6)-3'C18 is inactive. We did not observe any apparent reduction in virus production as compared with wild-type levels (Fig. 7b), which suggests that the compounds do not target the expression of the vector and virus assembly.

A neutralization assay was then performed to detect the compounds acting through inactivation of the viral particle. Recombinant NL4.3-Ren supernatants were incubated with 100nM of **GQVIR17-3'C18** for 3 h. Viral supernatants were next used to infect SupT1 cells diluting the virus to the final infectious titter, which in our experiments was 30 times. The drug present in the supernatant was therefore also diluted below EC₅₀ levels, e.g. 3nM. To ensure that replication inhibition was not due to residual activity, a control of dilution was used in which SupT 1 cells were infected with NL4.3-Ren in the presence of the final concentration of the dilution control (3nM). Inhibition of direct infection without neutralization treatment was also performed as a positive control (100nM). As shown in Fig. 7c, GQVIR17-3'C18 acts as a neutralizing agent, since 100nM concentration was capable of neutralizing 50% of the recombinant NL4.3-Ren. As expected, such a persistent effect suggests a direct binding of GQVIR17-3'C18 to the virus. To address this issue, we tested the effect of the compounds on HIV recombinant virus pseudo-typed with the G protein of VSV (NL4.3-deltaenv-VSV-Luc), which enters host cells independently of cell receptors. As shown in Fig. 7d, VSV-pseudotyped virus infection was not affected by the presence of neither GQVIR17-3'C18 nor seqVIR17(A-4)-3'C18, while 100 nanoM of GQVIR17-3'C18 protected at 70% the SupT1 cells form HIV NL4.3. Again, the seqVIR17(A4-6)-3'C18 did not show any effect. These results indicate that the GQVIR17-3'C18 activity is linked with HIV EnvGP and the molecule of the invention are virus entry inhibitors.

### 3. Influence of the membrane anchors on compounds activity

In order to evaluate the activity of the G4-ONs conjugates as a function of the lipophilic anchor, we compared the antiviral activity of GQVIR17, GQVIR17-3'C18 and GQVIR17-3'Chol on NL4.3-Ren (assay A, Fig. 8a) and HIV-1 Lai (assay B, Fig. 8b). In the assay A, we tested the fluorescent compounds 5'Cy5-GQVIR17-3'C18. The EC₅₀ obtained in both assays confirm the previous results: in assay A, EC₅₀=2µM for GQVIR17, EC₅₀=50nM for 5'Cy5-GQVIR17-3'C18 and GQVIR17-3'Chol; in assay B, EC₅₀=206nM for GQVIR17-3'C18 and EC₅₀=106nM for GQVIR-3'Chol. Cell viability was not affected with these new compounds. The antiviral activity of the G4-ON was not affected by the 5' addition of Cy5, and even increased by a factor 2. This last result suggests that such a compound could be used for fluorescent labelling of virus particles, a property eventually useful to design a virus titration assay based on fluorescence. Both assays show a 2-fold boost in the antiviral activity when a cholesterol group substituted the C18 alkyl chain. This shows that the antiviral activity of our G4-lipid conjugates can be tailored with membrane anchors of different nature.

In conclusion, the GQVIR17 sequence, only when structured into a G4-ON structure, shows a moderate antiviral effect against HIV-1. A 5x fold increased in the antiviral activity was observed when the GQVIR17 sequence, or a shorter GQVIR11, where functionalized in 3' with a membrane anchor such as a C18 alkyl chain or a cholesterol. The compounds do not alter cell viability and interfere with virus entry, as expected.

### Example 3: Antiviral activity of the compounds against HCV

### 1. GQVIR17-3'C18 inhibits HCVcc infection

To assess the inhibitory effects of GQVIR17-3'C18 on HCV infection we used the HCV cell-culture (HCVcc) system (Lindenbach et al., 2005, Science 309: 623-6). Our experiments were performed in the context of genotype 2a (Jc1 chimera HC-J6CH E2, Acc. No. AF177036) HCVcc virus. To facilitate the quantification of infection, we utilized the bicistronic Jc1 luciferase reporter construct, designated Luc-Jc1, in our infection analyses (Koutsoudakis et al., 2006, J Virol 80:5309-20). Cell viability was followed in parallel. The tyrosine kinase inhibitor Dasatinib, which is a known HCV entry inhibitor, was used as positive control.

As shown in Fig. 9, Luc-Jc1 virus infection was completely inhibited by GQVIR17-3'C18 at concentrations in the 5-10 µM range. Viability assays show an absence of toxicity of the compounds at 10 µM. The EC₅₀ for GQVIR17-3'C18 was estimated to be approx. 0.84 µM, as compared with an EC₅₀ of 1.47µM for Dasatinib in this assay (CC₅₀:20.31µM, SI:13.81). Conclusively, there is a strong inhibition of HCV infection by GQVIR17-3'C18, which is non-toxic at 10µM for the cultured human hepatoma cell line Huh7/Scr. To demonstrate the specific inhibitory activity of GQVIR17-3'C18 against HCV, similar experiments with increasing doses of the monomer structure seqVIR17(A4-6)-3'C18 were performed. As shown in Fig. 9c, and as seen with HIV, GQVIR17-3'C18 presented an approx. 7.6-fold lower EC₅₀than seqVIR17(A4-6)-3'C18 (0.51 µM versus 3.93 µM), indicating the role of the specific G4-ON structure in HCV neutralization.

### 2. GQVIR17-3'C18 inhibits HCVpp infection

HCV pseudoparticles (HCVpp) are generated by incorporation of the full-length HCV glycoproteins E1 and E2 into the envelope of lenti- or retroviral core particles. As has been validated by many research groups, HCVpp closely mimic the functionality of the wild-type virus in terms of cell entry and neutralization. Thus, we have assayed the effect of GQVIR17-3'C18 on an assay using HCVpp which carry identical EnvGP (genotype 2a, isolate J6CH) to the Luc-Jc1 viruses. The day of infections, Huh7/Scr cells were treated with 5 µM of GQVIR17-3'C18 or Dasatinib for 1 h. Then, compounds-containing media were removed and cells were infected with HCVpp-compounds mix at the same concentration. HCVpp-compounds mix were replaced 6 h post infection with fresh media-compounds mix and 72 h after infection cells were assayed for Renilla luciferase activity. The results are presented in Fig. 10, and show that GQVIR17-3'C18 inhibit HCVpp infection as previously seen with HCVcc. This demonstrates that GQVIR17-3'C18 exerts its inhibitory capacity during HCV entry. Similar to the HCVcc experiments, the compounds were found to be non-toxic at a concentration of 5 µM in Huh7/Scr cells.

### 3. GQVIR17-3'C18 do not inhibit HCV translation and/or replication

To investigate the impact of GQVIR17-3'C18 on HCV translation and/or replication, we transfected Huh7/Scr cells with a sub-genomic JFH1 luciferase replicon (Kato et al, 2003) and monitored RNA replication 48 h later by luciferase assays. As control we incubated the cells with increasing doses of the NS3-4A protease inhibitor telaprevir (VX-950). As shown in Fig. 11, GQVIR17-3'C18 affected neither RNA translation nor replication of HCV RNA because luciferase activity was not changed up to a dose of 10 µM.

### 4. GQVIR17-3'C18 interacts with HCV and inhibit early entry steps

To resolve which step of HCV cell entry is inhibited by GQVIR17-3'C18, we assessed their anti-HCV activity in time-of-addition experiments. To this end, Huh7/Scr cells were pre-incubated with GQVIR17-3'C18 for 1h prior to inoculation, or viruses were pre-incubated for 1h with GQVIR17-3'C18 prior to inoculation, or GQVIR17-3'C18 were added to virus-cells mix simultaneously to infection or 4 hours post inoculation (Fig. 12a). As shown in Figure 12b, and as seen with HIV-1, GQVIR17-3'C18 interfere with HCV infection only when they are pre-incubated with HCV viruses or added simultaneously to cells with the viruses, indicating a role of GQVIR17-3'C18 to the viral particles and/or initial steps of HCV entry. Further, in order to test if GQVIR17-3'C18 inhibits HCV attachment to the surface of target cells, Huh7/Scr cells were incubated with Jc1 virus without reporters in the presence or absence of inhibitors for 2 h at 4°C. Under these conditions, virus attaches to the cells but does not efficiently enter. Heparin sodium salt, which is known to inhibit HCV entry at the attachment step was used as a positive control. After 2h, viruses were removed, cells were washed extensively to remove the unbound virus and the bound HCV was quantified with RT-qPCR. As shown in Fig. 12c, GQVIR17-3'C18 efficiently inhibited HCV attachment, like Heparin, under these conditions, indicating a role at least in early entry steps.

HCV treatment efficacy is influenced by viral genotype. As specified above, interferon-based therapy regimes attain SVR rates of approx. 80% in patients with genotype 2 and 3 infections, while SVR rates among patients chronically infected with genotype 1 viruses drop to 50%. Moreover, HCV glycoproteins exert quite a heterogeneity, which is more profound in specific E2 regions, designated hyper-variable regions. Therefore, we determined the antiviral activity of GQVIR17-3'C18 toward HCV particles from different genotypes. To this end, we utilized the recently described JFH-1 based reporter virus constructs, carrying Renilla luciferase inserted at the NS5A gene and structural proteins from all major HCV genotypes: 1a (isolate TN), 1b (isolate J4), 2b (isolate J8), 3a (isolate S52), 4a (isolate ED43), 5a (isolate SA13), 6a (isolate HK6a) and 7a (isolate QC69) (Gottwein et al., 2011, J Virol 85:8913-28) (Fig. 13). Importantly, GQVIR17-3'C18 showed antiviral activity against all major genotypes, and the estimated EC₅₀ for each genotype (Table 2) was comparable to the EC₅₀ estimated previously for the genotype 2a HCVcc, indicating that GQVIR17-3'C18 inhibits HCV cell entry independently of viral genotype or subtype.

**Table 2: Antiviral activity (EC₅₀) of GQVIR17-3'C18 across all major HCV genotypes.**

| **GQVIR17-3'C18** | |
|---|---|
| **HCV genotype** | **EC₅₀ (µM)** |
| 1a (TN) | 0.93 |
| 1b (J4) | 0.23 |
| 2b (J8) | 0.34 |
| 3a (S52) | 0.27 |
| 4a (ED43) | 0.86 |
| 5a (SA13) | 1.18 |
| 6a (HK6a) | 1.51 |
| 7a (QC69) | 0.53 |

### 6 Basic residues of hypervariable region 1 (HVR1) and HVR1 itself play a role in GQVIR17-3'C18 mediated HCV neutralization.

The EnvGP of HCV exhibits a high degree of genetic heterogeneity, especially in the HVR1 of E2. However, positively-charged amino acids appear to be predominant in several HVR1 positions and may contribute to virus entry, for example, in the step of virus attachment to cellular anionic polysaccharides. To investigate the role of HVR1 basic amino acids in GQVIR17-3'C18-mediated HCV neutralization, we performed inhibition experiments using Luc-Jc1 wild-type (WT) viruses, a mutant that lacks HVR1 (ΔHVR1), and a mutant that possesses alanines instead of basic amino acids in the HVR1 region (basic-). As shown in Figure 14, at a final concentration of 1 µM, WT viruses were inhibited up to ∼80%, while both mutants basic- and ΔHVR1 were resistant, suggesting a direct role of basic amino acids of HVR1 and/or of the HVR1 region itself in GQVIR17-3'C18 antiviral activity.

In conclusion, the lipid-G4-ON conjugates of the invention inhibit major phenotypes of HCV at µM concentration, with low cytotoxicity for huh7 cells. This effect affects HCV entry or attachment, is dependent on E2 protein and more precisely on a patch of basic amino-acids residues in the HRV1, which suggests a non-specific binding of the G4-ON to at least the E2 EnvGP of HCV.

Moreover, it is shown herein that GQVIR17-3'C18 has antiviral activity against both HIV and HCV. This is, to our knowledge, the first reported case of a synthetic ODN with such a capacity.

### Example 4: Cellular localization of 5'Cy5-GQVIR17-3'C18

Fluorescence microscopy was used to analyse the location of the 5'Cy5-GQVIR17-3'C18 compound in HeLa cells. Cell viability was followed in presence of increasing concentrations of the compound (Figure 15b). Figure 15a shows an accumulation of intense intracellular fluorescent vesicles and diffuse membrane labelling, without fluorescence in the cytoplasm. This has not been observed with other fluorescent G4-ONs incubated with cells. The compound was seen not toxic at this concentration (1 µM) and more (10 µM) for 48 h. The membrane distribution of the compounds was not seen homogenous but rather showed a clear segregation of fluorescence in relatively large patches of membranes and/or endocytosis vesicles. This suggests a local concentration of the compounds in certain membrane domains. This also suggests that the conjugates could bind directly to the viral particle, in accordance with their potent neutralization effect observed with HIV and HCV.

## Claims

1. An agent comprising one unit, two units or four units, wherein each of said units comprises:
(i) an oligonucleotide comprising at least four consecutive guanines (G), wherein said at least four consecutive G form an intra-unit (intra-molecular) or an inter-unit (inter-molecular) G-quadruplex, wherein said G-quadruplex binds to a viral envelope glycoprotein,
and wherein at least one of said units comprises:
(ii) a lipophilic substituent moiety, wherein said lipophilic molecule anchors to a viral envelope or to the membrane of a cell susceptible of being infected by a virus, and
(iii) a linker, wherein said linker links the 5' or 3' end of the oligonucleotide in (i) to the lipophilic substituent moiety in (ii),
wherein said agent has antiviral activity.

2. An agent according to claim 1, wherein the oligonucleotide in (i) comprises the nucleotide sequence
*5'- X₁ X₂ G G G (G)ᵢ X₃* (SEQ ID NO:8)*(tail) - 3'*
wherein
- X₁ may be present or absent, and represents any nucleotide,
- X₂ represents T, A or C, preferably T or A,
- i is an integer between 1 and 4,
- X₃ represents T, A or C, preferably T or A,
- (tail) represents a nucleotide sequence, comprising between 0 and 23 nucleotides.

3. An agent according to any of claims 1 or 2, wherein said agent comprises one unit, and wherein said unit is arranged in a monomolecular G-quadruplex.

4. An agent according to claim 3, wherein the oligonucleotide tail comprises three repetitions of at least three consecutive G, wherein each of said at least three consecutive G is separated from the following at least three consecutive G by at least one nucleotide.

5. An agent according to any of claims 1 or 2, wherein said agent comprises two units, and wherein said two units are arranged in a bimolecular G-quadruplex.

6. An agent according to claim 1 or 2, wherein said agent comprises four units, and wherein said four units are arranged in a tetramolecular parallel G-quadruplex.

7. An agent according to claim 6, wherein the oligonucleotide tail does not comprise at least two consecutive G.

8. An agent according to any of claims 6 or 7, wherein the oligonucleotide comprises the sequence 5'-TTGGGGGGTAC-3' (SEQ ID NO: 2).

9. An agent according to claim 8, wherein the oligonucleotide comprises the sequence 5'-TTGGGGGGTACAGTGCA-3' (SEQ ID NO: 3).

10. An agent according to any of claims 1 to 9, wherein the lipophilic substituent moiety is a lipid selected from the group consisting of a fatty acid, an acylglyceride, a phospholipid, a glucolipid, a terpenoid and a steroid, or comprises a C8-C30 alkyl substituent.

11. An agent according to any of claims 1 to 10, wherein the envelope glycoprotein is an envelope glycoprotein from an enveloped virus.

12. A pharmaceutical composition comprising a therapeutically effective amount of an agent according to any of claims 1 to 11, together with at least one pharmaceutically acceptable excipient or carrier.

13. An agent according to any of claims 1 to 11 or a pharmaceutical composition according to claim 12 for use in medicine.

14. An agent according to any of claims 1 to 11 or a pharmaceutical composition according to claim 12 for use in the prevention and/or treatment of viral infection in a subject, wherein said viral infection is caused by an enveloped virus.

15. An agent or a pharmaceutical composition for use according to claim 14, wherein the enveloped virus is selected from the group consisting of human immunodeficiency virus (HIV), a hepatitis virus selected from hepatitis A virus (HAV), hepatitis B virus (HBV), hepatitis C virus (HCV), hepatitis D virus (HDV), and hepatitis E virus (HEV), herpes simplex virus (HSV), Epstein-Barr virus (EBV), respiratory syncytial virus (RSV), human cytomegalovirus, human T-lymphotropic virus I (HLTV I), human T-lymphotropic virus II (HLTV II), West Nile virus, Dengue virus and Chikungunya virus.


SEQUENCE LISTING

<110>  INSTITUT D'INVESTIGACIONS BIOMÈDIQUES AUGUST PI I SUNYER
       (IDIBAPS)
       CONSEJO SUPERIOR DE INVESTIGACIONES CIENTÍFICAS
       CENTRO DE INVESTIGACIÓN BIOMÉDICA EN RED (CIBER)
       UNIVERSITAT POMPEU FABRA
       INSTITUCIÓ CATALANA DE RECERCA I ESTUDIS AVANÇATS

<120>  ANTIVIRAL AGENTS COMPRISING AN OLIGONUCLEOTIDE-LIPID CONJUGATE
       FORMING G-QUADRUPLEX

<130>  P7363EP00

<160>  8

<170>  PatentIn version 3.5

<210>  1
<211>  27
<212>  DNA
<213>  Artificial Sequence

<220>
<223>  Oligonucleotide

<400>  1
ttggggatag ggatagggat agggata                                          27


<210>  2
<211>  11
<212>  DNA
<213>  Artificial Sequence

<220>
<223>  Oligonucleotide

<400>  2
   ttggggggta c                                                            11


<210>  3
<211>  17
<212>  DNA
<213>  Artificial Sequence

<220>
<223>  GQVIR17

<400>  3
   ttggggggta cagtgca                                                      17


<210>  4
<211>  17
<212>  DNA
<213>  Artificial Sequence

<220>
<223>  GQVIR17(A4-6)

<400>  4

ttgaaaggta cagtgca                                                    17


<210>  5
<211>  24
<212>  DNA
<213>  Artificial Sequence

<220>
<223>  S-292 probe

<400>  5
cctgataggg tgcttgcgag tgcc                                           24


<210>  6
<211>  20
<212>  DNA
<213>  Artificial Sequence

<220>
<223>  S-271 primer

<400>  6
gcgaaaggcc ttgtggtact                                                20


<210>  7
<211>  20
<212>  DNA
<213>  Artificial Sequence

<220>
<223>  A-337 primer

<400>  7
cacggtctac gagacctccc                                                20


<210>  8
<211>  10
<212>  DNA
<213>  Artificial Sequence

<220>
<223>  Oligonucleotide


<220>
<221>  misc_difference
<222>  (1)..(1)
<223>  nucleotide in this position may be present or absent

<220>
<221>  misc_feature
<222>  (1)..(1)
<223>  n is a, c, g, or t

<220>
<221>  misc_difference
<222>  (7)..(7)
<223>  nucleotide in this position may be present or absent

<220>
<221>  misc_difference
<222>  (8)..(8)
<223>  nucleotide in this position may be present or absent

<220>
<221>  misc_difference
<222>  (9)..(9)
<223>  nucleotide in this position may be present or absent

<400>  8
nhgggggggh                                                                10